# EUROPEAN PATENT SPECIFICATION

(11) **EP 1 416 972 B1**
(45) Date of publication and mention of the grant of the patent: **08.02.2012**
(21) Application number: 01992062.8
(22) Date of filing: 09.11.2001
(51) Int. Cl.: A61K 48/00, C07K 17/10, C12N 15/63, C07K 17/02

(54) **CHIMERIC MOLECULES TO MODULATE GENE EXPRESSION**
CHIMÄRE MOLEKÜLE ZUR MODULATION DER GENEXPRESSION
MOLECULES CHIMERES SERVANT A MODULER L'EXPRESSION D'UN GENE

(30) Priority: 09.11.2000 US 304182 P
(43) Date of publication of application: 12.05.2004
(73) Proprietor: COLD SPRING HARBOR LABORATORY, Cold Spring Harbor, NY 11724 (US)
(72) Inventor: KRAINER, Adrian, R., Huntington Station, NY 11746 (US); CARTEGNI, Luca, Huntington Station, NY 11746 (US)
(74) Representative: Grünecker, Kinkeldey, Stockmair & Schwanhäusser
(86) International application number: PCT/US2001/047523
(87) International publication number: WO 2002/038738

(56) References cited:
- WO-A-95/01370
- US-B1- 6 265 388
- HYRUP B ET AL: "PEPTIDE NUCLEIC ACIDS (PNA): SYNTHESIS, PROPERTIES AND POTENTIAL APPLICATIONS" BIOORGANIC & MEDICINAL CHEMISTRY, ELSEVIER SCIENCE LTD, GB, vol. 4, no. 1, 1996, pages 5-23, XP000602327 ISSN: 0968-0896
- MAPP A K ET AL: "Activation of gene expression by small molecule transcription factors." PROCEEDINGS OF THE NATIONAL ACADEMY OF SCIENCES OF THE UNITED STATES OF AMERICA. 11 APR 2000, vol. 97, no. 8, 11 April 2000 (2000-04-11), pages 3930-3935, XP002289613 ISSN: 0027-8424
- LORSON C L ET AL: "A single nucleotide in the SMN gene regulates splicing and is responsible for spinal muscular atrophy." PROCEEDINGS OF THE NATIONAL ACADEMY OF SCIENCES OF THE UNITED STATES OF AMERICA. 25 MAY 1999, vol. 96, no. 11, 25 May 1999 (1999-05-25), pages 6307-6311, XP002153155 ISSN: 0027-8424
- COOPER THOMAS A ET AL: "The regulation of splice-site selection, and its role in human disease" AMERICAN JOURNAL OF HUMAN GENETICS, UNIVERSITY OF CHICAGO PRESS, CHICAGO,, US, vol. 61, no. 2, 1997, pages 259-266, XP002204084 ISSN: 0002-9297
- MAYEDA A ET AL: "Substrate specificities of SR proteins in constitutive splicing are determined by their RNA recognition motifs and composite pre-mRNA exonic elements." MOLECULAR AND CELLULAR BIOLOGY. MAR 1999, vol. 19, no. 3, March 1999 (1999-03), pages 1853-1863, XP002289614 ISSN: 0270-7306
- LIU H X ET AL: "Exonic splicing enhancer motif recognized by human SC35 under splicing conditions." MOLECULAR AND CELLULAR BIOLOGY. FEB 2000, vol. 20, no. 3, February 2000 (2000-02), pages 1063-1071, XP002289615 ISSN: 0270-7306
- BLENCOWE B J B J: "Exonic splicing enhancers: mechanism of action, diversity and role in human genetic diseases" TIBS TRENDS IN BIOCHEMICAL SCIENCES, ELSEVIER PUBLICATION, CAMBRIDGE, EN, vol. 25, no. 3, March 2000 (2000-03), pages 106-110, XP004202539 ISSN: 0968-0004
- CARTEGNI L ET AL: "Correction of disease-associated exon skipping by synthetic exon-specific activators" NATURE STRUCTURAL BIOLOGY, NEW YORK, NY, US, vol. 10, no. 2, February 2003 (2003-02), pages 120-125, XP002262731 ISSN: 1072-8368
- CARTEGNI LUCA ET AL: "Disruption of an SF2/ASF-dependent exonic splicing enhancer in SMN2 causes spinal muscular atrophy in the absence of SMN1." NATURE GENETICS. APR 2002, vol. 30, no. 4, April 2002 (2002-04), pages 377-384, XP002289616 ISSN: 1061-4036
- LIU H X ET AL: "A mechanism for exon skipping caused by nonsense or missense mutations in BRCA1 and other genes." NATURE GENETICS. JAN 2001, vol. 27, no. 1, January 2001 (2001-01), pages 55-58, XP002289617 ISSN: 1061-4036

## Description

This work was supported by the following grants: GM42699 and CA13106 from the N.I.H. The government has certain rights to this invention.

This application asserts the priority of provisional U. S. application 60/3 04,182 filed November 9, 2000

### BACKGROUND OF THE INVENTION

Gene expression is the process by which the protein product of a gene is made. Included in gene expression are the steps of transcription, splicing and translation. Transcription is the process by which information from double-stranded DNA is converted into its single-stranded RNA equivalent, termed a pre-mRNA transcript. Splicing is the process by which introns of the pre-mRNA transcript are removed; and the remaining exons are joined to form mRNA. Translation is the synthesis of a protein using the mRNA as a template.

The ability to modulate gene expression is a valuable tool both for research and therapeutic purposes. For example, a researcher may wish to modulate the activity of a particular gene so as to identify the function of the gene, the effect the gene product's cellular concentration has on the function of the cell, or other cellular characteristics. With respect to therapeutics, one may wish to modulate gene expression in order to increase the production of certain proteins that may not be produced, or are produced at low levels, by the native gene. The proteins may not be produced at sufficient levels due to a disease state or a genetic mutation.

Attempts have been made to modulate gene expression at the level of transcription. For example, Dervan et al. describe an artificial transcription factor. (Dervan et al., PNAS 97: 3930-3935.) The factor consists of a DNA-binding polyamide tethered to a peptide transcriptional activation domain. The polyamide contains a total of eight *N*-methylimidazole and *N*-methylpyrrole amino acids in the form of a hairpin structure. This structure results in the amino acids being side-by-side to form four pairs. The possible pairing types described are an imidazole paired with a pyrrole, and a pyrrole paired with a pyrrole.

The polyamide binds to the minor groove of a DNA molecule via hydrogen bonds. The DNA-binding specificity depends on the type of the amino acid pairing. A pairing of imidazole opposite pyrrole targets a G·C base pair, whereas pyrrole opposite imidazole targets a C·G base pair. A pyrrole/pyrrole combination is degenerate and targets both T·A and A·T base pairs.

The method for modulating gene expression described by Dervan et al. has several limitations. For example, the DNA-binding hairpin polyamides described by Dervan et al. contain eight amides. Accordingly, these polyamides can be inserted between four nucleic acid base pairs of a DNA molecule. A series of such a length is too short to allow for binding of high specificity. For example, a series of at least ten to twenty bases are necessary in order to target a unique natural DNA sequence in prokaryotes and eukaryotes. Seventeen to eighteen bases are necessary to target a unique sequence in the human genome.

In addition to the insufficient length of the Dervan et al. polyamides, binding of these polyamides are not as precise as would result from Watson-Crick base-pairing. For example, the polyamides cannot distinguish between AT and TA base pairs. This degeneracy further decreases the specificity by which the Dervan et al. polyamides can bind to DNA.

Another limitation in the method of Dervan et al. is that the binding polyamides can only bind to double-stranded DNA. However, the modulation of splicing and translation both involve single-stranded RNAs. Accordingly, transcription is the only step of gene expression that can be modulated by the method ofDervan et al. Splicing and translation cannot be modulated by the method of Dervan et al.

Another attempt to modulate gene expression at the level of transcription is disclosed by Ecker et al. (U.S. Patent No.: 5,986,053). In particular, Ecker et al. disclose "conjugates" which are peptide nucleic acids (PNAs) conjugated to proteins. The proteins are transcription factors.

The method for modulating gene expression described by Ecker et al. has several limitations. For example, since transcription factors contain anywhere from about one hundred fifty to over a thousand residues, the "conjugates" disclosed by Ecker et al. are difficult to synthesize. The length of these "conjugates" also renders *in vivo* delivery and cellular uptake difficult. Consequently, the value of these "conjugates" as therapeutic agents is questionable.

Another limitation of the method of Ecker et al. for modulating gene expression is that the only modulation contemplated is at the level of transcription. Ecker et al. does not address the splicing and translation steps of gene expression.

The object of the present invention is to provide molecules that modulate splicing and/or translation. Additionally, the object of the invention is to modulate transcription with molecules which bind with high specificity to double-stranded nucleic acid molecules and which provide ease of synthesis and delivery.

### SUMMARY

These and other objects, as would be apparent to those skilled in the art, have been achieved by providing chimeric molecules which comprise a base-pairing segment comprising naturally-occuring or modified purine and/or pyrimidine bases joined to a polypeptide moiety comprising a domain comprising dipeptide repeats, wherein the base-pairing segment binds specifically to a segment of pre-mRNA according to Watson-Crick rules of base pairing, and the polypeptide moiety modulates splicing of the segment of pre-mRNA. In one embodiment, the invention relates to an in vitro method for modulating splicing and translation. The method comprises contacting a single-stranded nucleic acid molecule with the chimeric molecule whereby the binding of the base-pairing segment allows the moiety to modulate splicing and translation. Further disclosed herein is a method to correct defective splicing of a pre-mRNA transcript during pre-mRNA splicing. The method comprises contacting the pre-mRNA transcript with the chimeric molecules whereby the binding of the base-pairing segment allows the moiety to correct defective splicing.

Further disclosed herein is a chimeric molecule which comprises a base-pairing segment that binds specifically to a double-stranded nucleic acid molecule, and a peptide that modulates transcription, wherein the peptide comprises up to about one hundred amino acid residues. Further disclosed herein is a method for modulating transcription. The method comprises contacting a double-stranded nucleic acid molecule with the chimeric molecule, whereby the binding of the base-pairing segment allows the peptide to modulate transcription.

Further disclosed herein is a method of making chimeric molecules that modulate gene expression. The method comprises covalently bonding a base-pairing segment that binds specifically to a nucleic acid molecule, and a moiety that modulates gene expression.

### BRIEF DESCRIPTION OF THE FIGURES

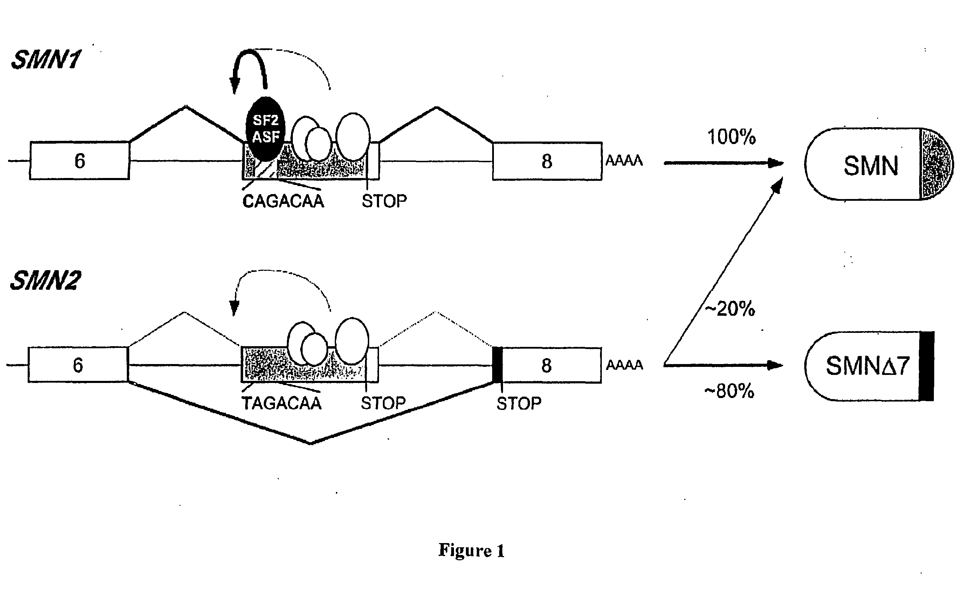

**Figure 1** is a model of SF2/ASF-dependent exon 7 inclusion in *SMN1* and *SMN2.* Binding of SF2/ASF to its cognate heptamer ESE in *SMN1* exon 7 (top) promotes exon definition, such that exon 7 is constitutively included, allowing for translation of full-length SMN protein. The C6T change in *SMN2* exon 7 (bottom) prevents efficient SF2/ASF binding to the corresponding heptamer. Exon 7 is thus mostly skipped, resulting in the production of defective SMNΔ7 protein. Other ESEs in the exon can mediate weak exon inclusion even in the absence of the SF2/ASF motif, probably through binding of other SR or SR-like proteins, which may include hTra2β1. Partial inclusion of *SMN2* exon 7 generates a small amount of full-length SMN protein, identical to that encoded by the *SMN1* gene. Exons are represented as boxes and introns as lines. The gray box indicates a region of exon 7 encoding the last 16 amino acids of the SMN protein, which are missing from SMNΔ7. The dark box in exon 8 represents the last four amino acids of SMNΔ7, which are not present in SMN. Open boxes represent 3' untranslated regions. The hatched box in *SMN1* exon 7 marks the position of the SF2/ASF heptamer ESE. The corresponding heptamer is indicated below *SMN2* exon 7, with position 6 in bold. The dark oval denotes SF2/ASF and open ovals represent SR or SR-like proteins. Arrows denote promotion of exon definition and chevrons indicate splicing patterns. Line thicknesses are indicative of relative splicing efficiency. The percent values refer to the extent of exon 7 inclusion *in vivo.* The diagrams of SMN and SMNΔ7 proteins illustrate the different C-terminal domains. For simplicity, other SMN isoforms are not considered in this model. Drawings are not to scale.

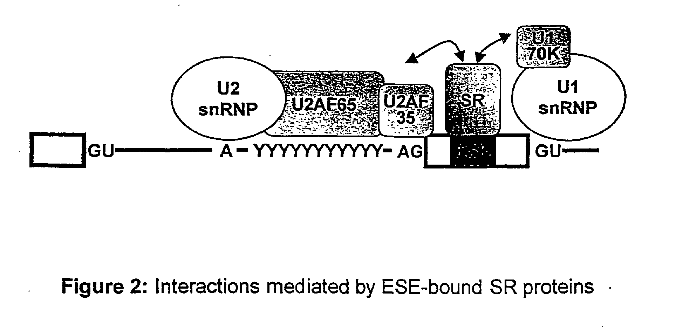

**Figure 2** is a diagram showing theoretical interactions mediated by ESE-bound SR proteins. ESE-bound SR proteins participate in protein-protein interactions to recruit spliceosome components to the adjacent intron elements during the earliest stages of spliceosome assembly. For example, the RS domain of SR proteins is thought to contact the RS domain of U2AF³⁵, indirectly facilitating binding of the large U2AF subunit, U2AF⁶⁵, to the 3' splice site poly-pyrimidine tract. U2AF⁶⁵, in turn, is known to facilitate binding of the U2 snRNP to the branch site via base pairing between U2 snRNA and the branch site element. SR proteins bound to exonic enhancers are also thought to facilitate binding of U1 snRNP at the downstream 5' splice site, except in the case of 3' terminal exons, for which an interplay between splicing and 3' end processing has been well documented. All these interactions are part of the process of exon definition, by which spliceosomal components initially identify exon-intron boundaries correctly, despite the very large size of some introns and the degeneracy of the splice site signals. The interaction between SR proteins and U1 snRNP again appears to be mediated by the SR protein RS domain, and, on the U1 snRNP side, by a related domain present in the 70K polypeptide.

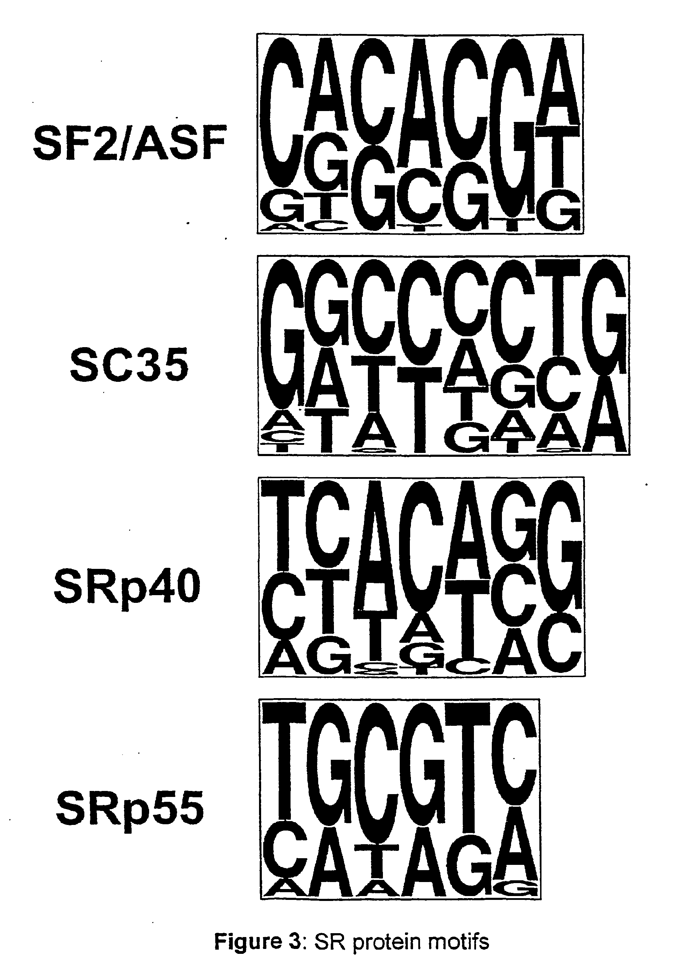

**Figure 3** is a diagram showing the motifs recognized by four SR proteins, displaying each nucleotide with a size proportional to its frequency at that position of the consensus. These motifs define sequences that function as exonic splicing enhancers in the presence of the cognate SR protein.

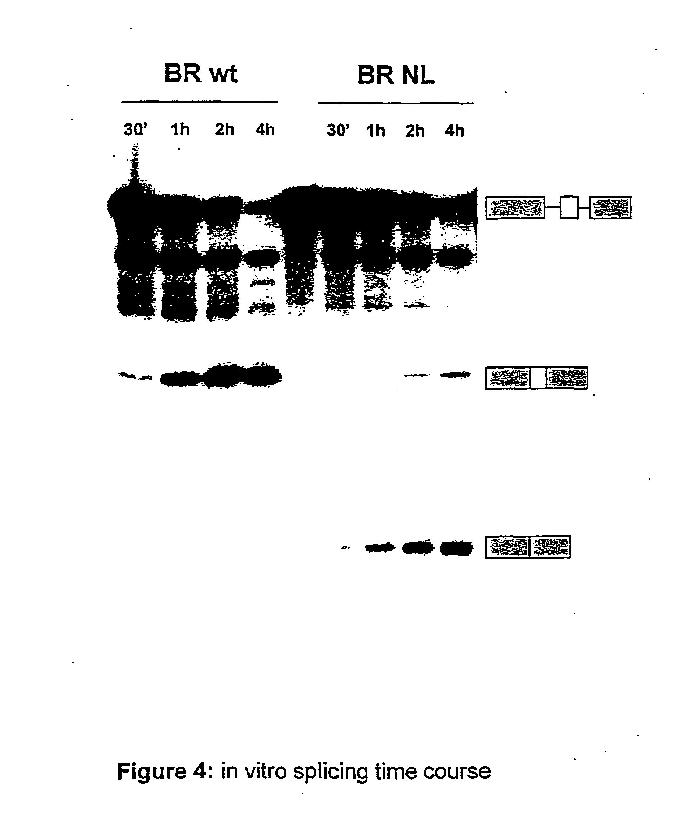

**Figure 4** shows the time course results of an *in vitro* splicing assay using a three-exon minigene and shortened versions of the introns *of BRCA1.* Splicing of the wild type (BR wt) and mutant (BR NL) transcripts in HeLa nuclear extract reproduced the *in vivo* effect of the mutation on exon 18 inclusion.

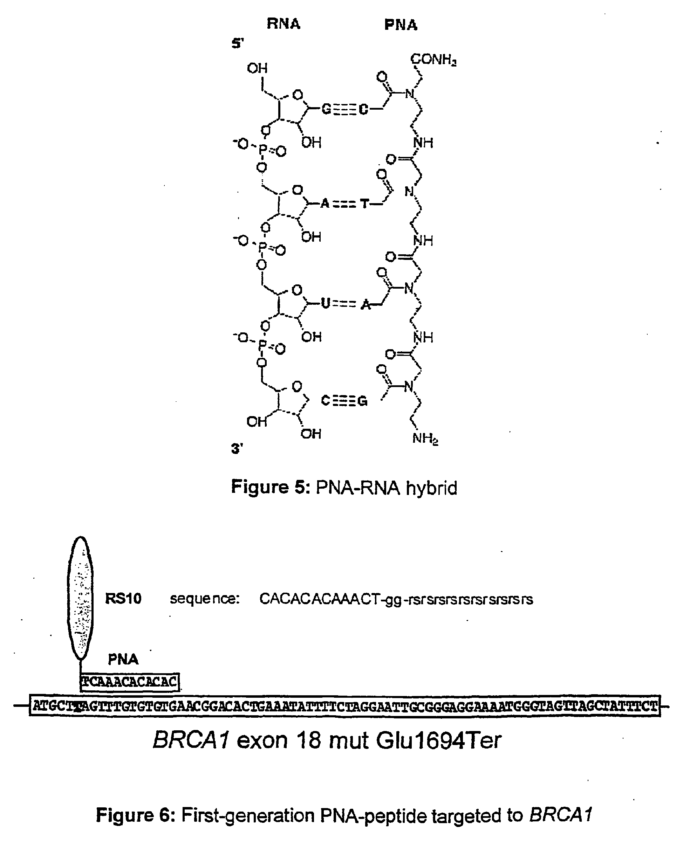

**Figure 5** shows a structural representation of a PNA-RNA hybrid.
**Figure 6** is a diagram showing a PNA-peptide targeted to *BRCA1* pre-mRNA transcript. The PNA is positioned one nucleotide downstream of the mutation at exonic position +6 in *BRCA1* exon 18, so it can hybridize equivalently to wild-type and mutant sequences.

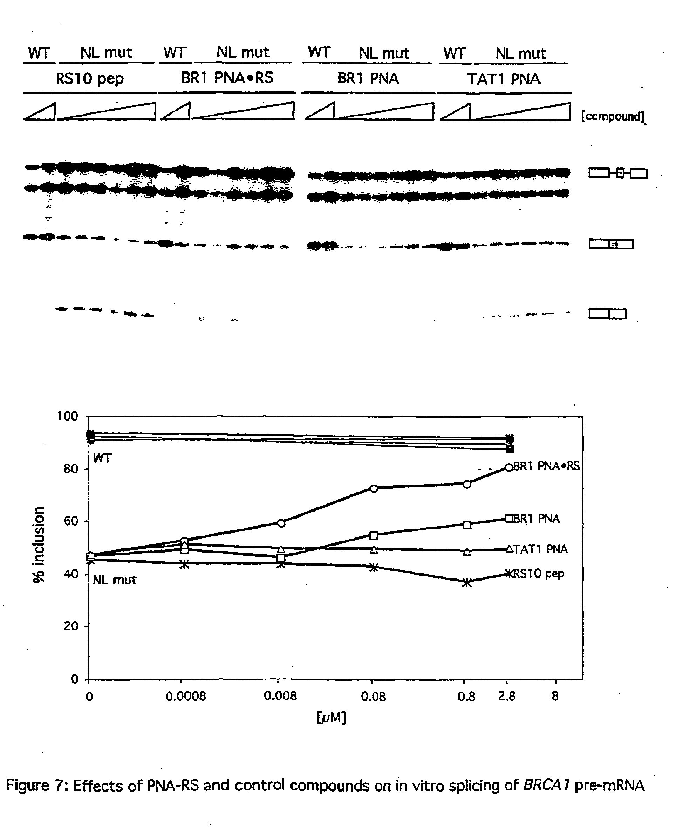

**Figure 7** shows effects of PNA-RS and control compounds on *in vitro* splicing of *BRCA1* pre-mRNA. The products of splicing were analyzed by denaturing PAGE and autoradiography (top). The percentage of exon 18 inclusion was quantitated (bottom); the points on the curves are open symbols for the mutant, and solid symbols for the wild-type. The dose-response curves for each compound show that the PNA-peptide (BR PNA·RS) was effective at promoting exon 18 inclusion with pre-mRNA harboring the patient nonsense mutation at position +6 (NL mut).

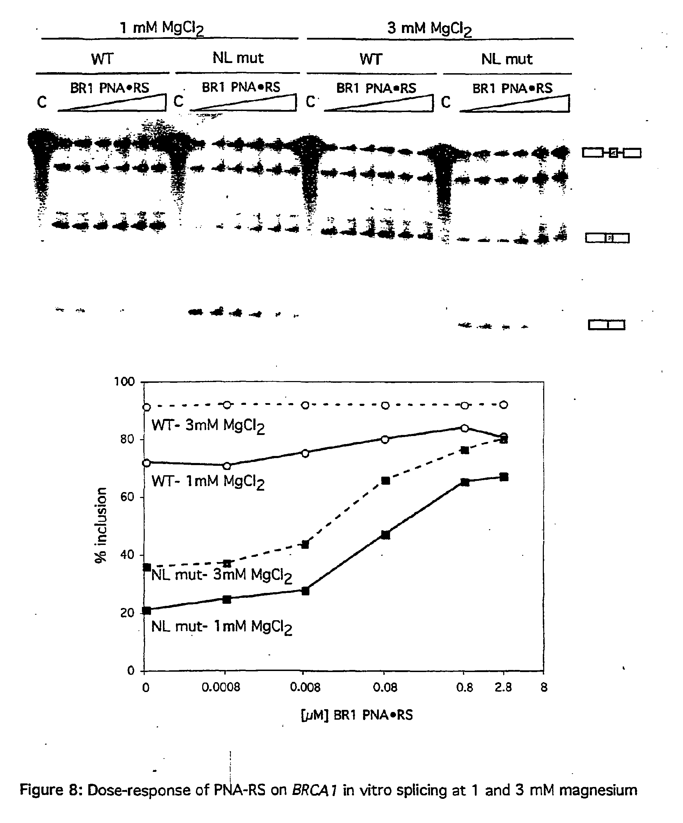

**Figure 8** shows the dose-response of PNA-RS on *BRCA1 in vitro* splicing at 1 and 3 mM magnesium. The C lanes show the input pre-mRNAs.

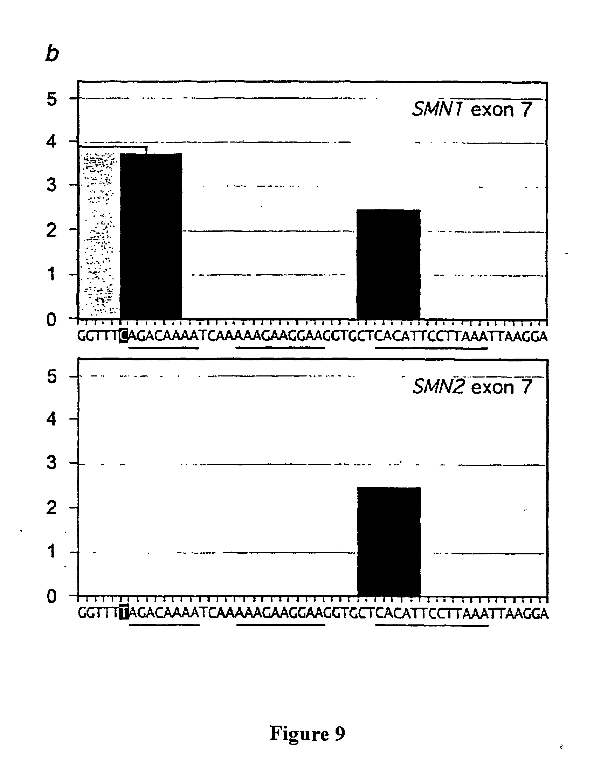

**Figure 9** is a graph showing the SR protein motif distribution in *SMN1* and *SMN2* exon 7. The 54-nt sequence of exon 7 in *SMN1* (top) and *SMN2* (bottom) was searched with four nucleotide-frequency matrices derived from pools of functional enhancer sequences selected iteratively *in vitro.* Motif scores reflect the extent of matching to a degenerate consensus, adjusted for background nucleotide composition, and only the scores above the threshold for each SR protein are shown. Gray and black bars represent SC35 and SF2/ASF high-score motifs, respectively. No SRp40 or SRp55 high-score motifs are present in exon 7. The height of each bar indicates the score value, the position along the x axis indicates its location along the exon, and the width of the bar represents the length of the motif The C at position +6 in *SMN1* is highlighted. The T at the same position in *SMN2* causes both SF2/ASF and SC35 scores to fall below threshold (3.76 to 0.81 and 3.87 to 2.14, respectively). Thresholds and maximal values are different for different SR proteins. The horizontal lines below the exon sequence mark the locations of putative exonic splicing enhancers (SE1, SE2, and SE3, respectively).

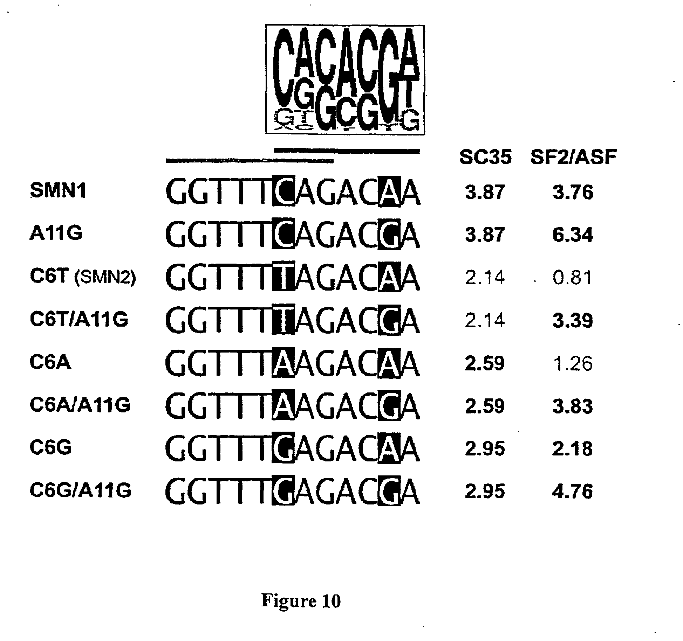

**Figure 10** is a graph showing the effect of point mutations on calculated SC35 and SF2/ASF motif scores. The first 12 nucleotides of exon 7 are shown, with the mutated positions +6 and +11 highlighted. The gray and black horizontal bars indicate the position of the SC35 and SF2/ASF motifs, respectively. The SF2/ASF consensus heptamer motif is aligned at the top. The effect of the point mutations used in transfection experiments on the calculated SC35 and SF2/ASF motif scores is shown on the right (high scores in black; sub-threshold scores in gray).

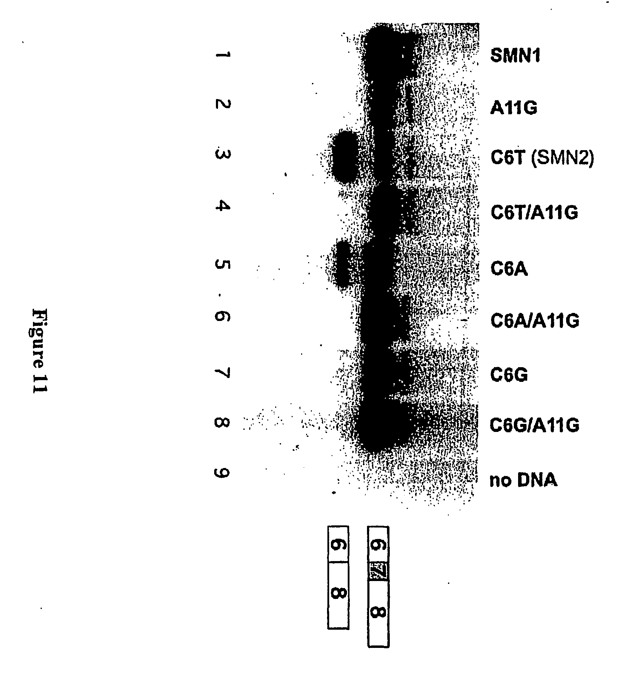

**Figure 11** illustrates that exon 7 skipping correlates with disruption of the proximal SF2/ASF heptamer motif Semi-quantitative [α-³²P] dATP-labeled RT-PCR analysis of transient expression of *SMN* minigenes. The products corresponding to exon 7 skipping and inclusion are indicated. The A11G suppressor mutation that reconstitutes an SF2/ASF high-score motif (lanes 4 and 6) restores correct splicing when the mutation at position +6 causes exon skipping (lanes 3 and 5).

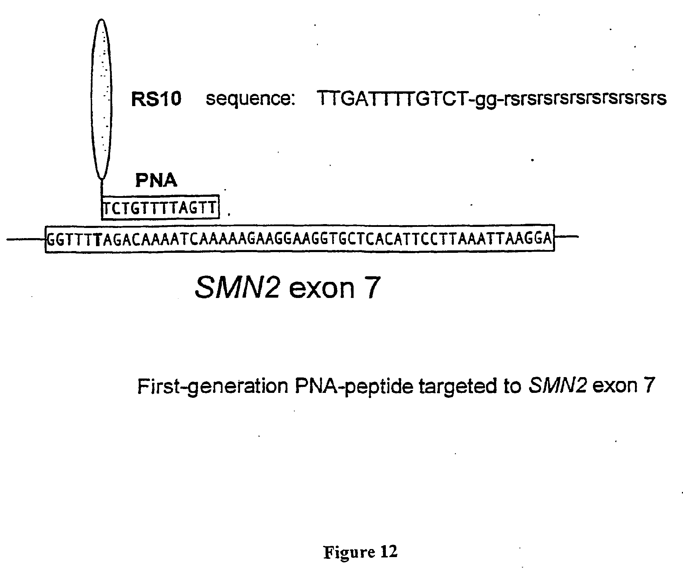

**Figure 12** is a diagram showing a PNA-peptide targeted to *SMN2* exon 7.

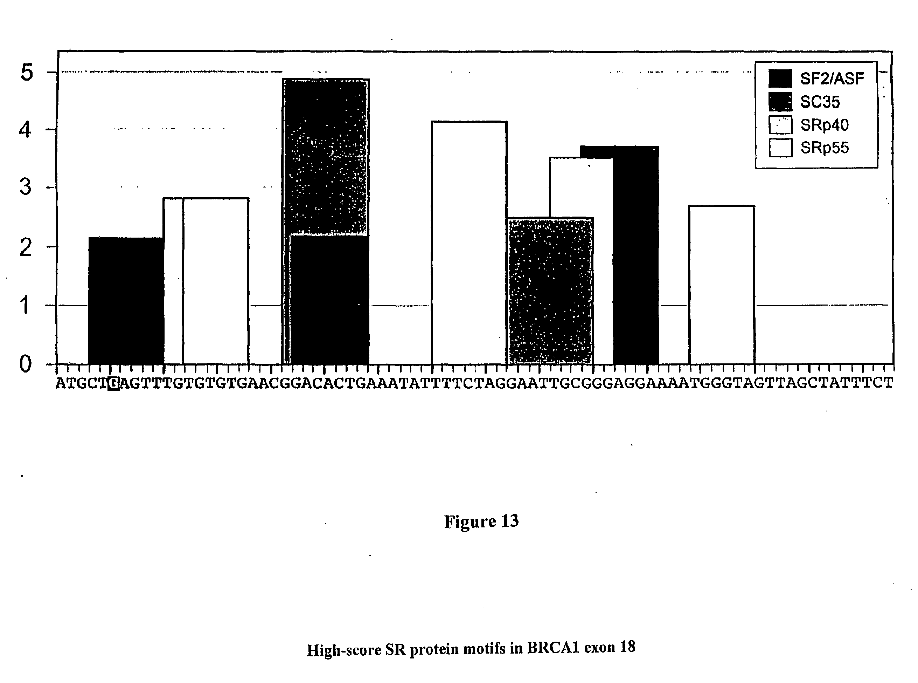

**Figure 13** is a graph showing the high-score SR protein motifs in *BRCAI* exon 18. Motif scores reflect the extent of matching to a degenerate consensus, and only the scores above the threshold for each SR protein are shown. High-score motifs are shown in black for SF2/ASF, dark grey for SC35, light grey for SRp40, and white for SRp55. The width of each bar reflects the length of the motif (6, 7, or 8 nt), the placement of each bar along the x axis indicates the position of a motif along the wild-type exon DNA sequence, and the height of the bar shows the numerical score on the y axis.

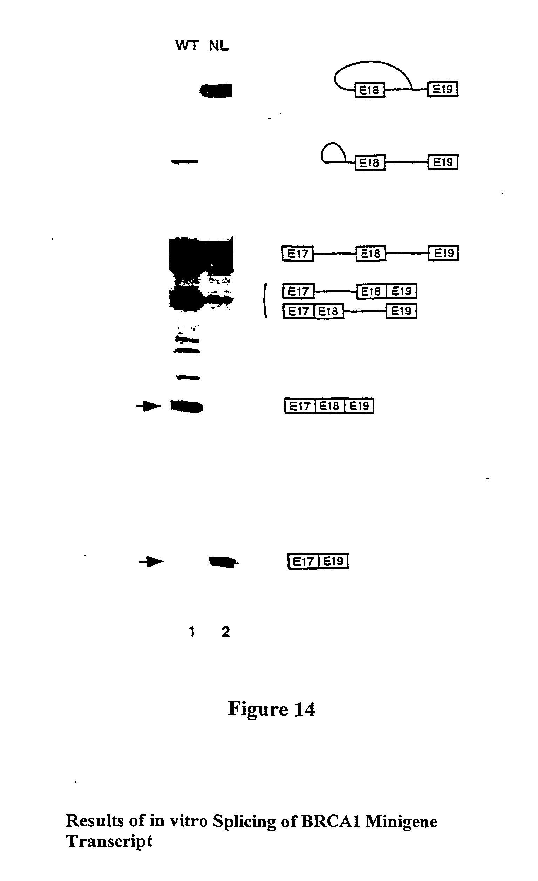

**Figure 14** shows the results of *in vitro* splicing of *BRCA1* minigene transcripts. The exon-skipping phenotype of a nonsense mutation is reproduced. Wild-type (WT, lane 1) and nonsense mutant with low SF2/ASF score (NL, lane 2) radiolabeled transcripts were spliced in HeLa cell nuclear extract, and the products of the reaction were analyzed by denaturing PAGE and autoradiography. The identity of each band is indicated schematically on the right. Exons 17 and 19 are shown as grey boxes, exon 18 as a white box, and the shortened introns as lines. The arrows indicate the mRNAs generated by exon 18 inclusion or skipping.

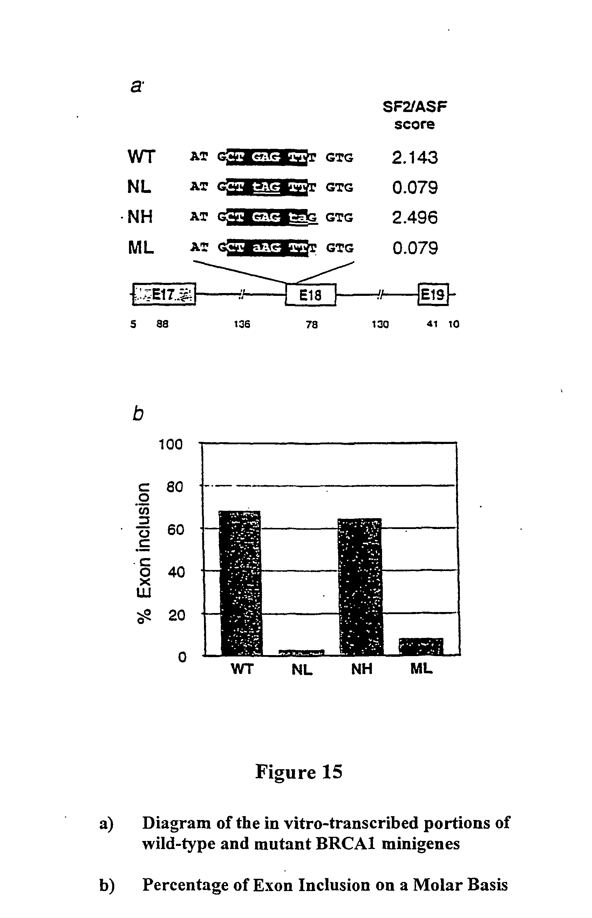

**Figure 15** illustrates that exon skipping correlates with the SF2/ASF enhancer motif score and not with reading frame disruption. Figure 15a shows a diagram of the *in vitro*-transcribed portions of wild-type and mutant *BRCA1* minigenes. The relevant portion of the exon 18 sequence is shown above the diagram, beginning at position 1 and with the triplet grouping indicating the reading frame. The heptamer sequence corresponding to the first SF2/ASF motif in Fig. 13 is highlighted. The mutated nucleotides are shown in lowercase, and the in-frame nonsense codons are underlined. WT - wild-type; NL - original nonsense mutant with a low SF2/ASF motif score; NH - nonsense mutant with a high score; ML - missense mutant with a low score. The calculated scores for the highlighted heptamers are shown on the right. The sizes of the exons and truncated introns, including 5 nt of T7 sequence and 10 nt of intron 19, are shown below the diagram. WT, NL, NH, and ML pre-mRNAs were spliced *in vitro* as in Fig. 14. The intensities of the mRNA bands arising from exon 18 inclusion or skipping were measured, and the percent inclusion on a molar basis was calculated and is shown in Figure 15b.

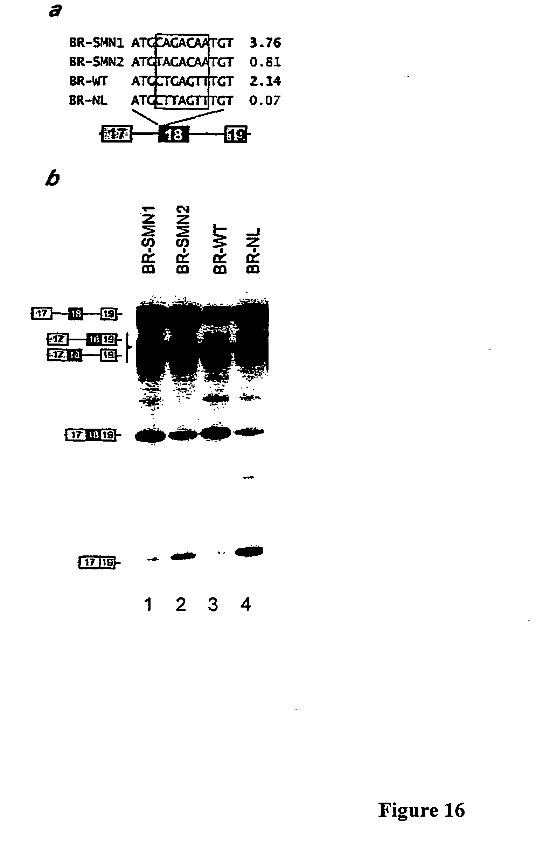

**Figure 16** illustrates the *SMN1* SF2/ASF heptamer motif is a *bona fide* ESE. a.) *BRCA1* minigenes used for *in vitro* transcription and splicing. The relevant portion of *BRCA1* exon 18 is shown above the diagram, starting with position +1 of each sequence. The calculated SF2/ASF motif scores corresponding to the highlighted heptamers are indicated for each minigene (high scores in black; sub-threshold scores in gray). The high-score SF2/ASF ESE in the *BRCA1* minigene (BR-WT) was replaced by the SF2/ASF heptamer from *SMN1,* or by the corresponding heptamer from *SMN2* (6CT). The pre-mRNA containing a natural *BRCA1* nonsense mutation (E1694X) that abrogates an SF2/ASF-dependent ESE (BR-NL) is also shown. b.) The SF2/ASF heptamer motif from *SMN1* promotes exon inclusion in a heterologous context (*BRCA1* exon 18). The four indicated *BRCA1*-derived pre-mRNAs were spliced in HeLa cell nuclear extract for 4 hours. The identity of each band is indicated schematically on the left. The sizes of pre-mRNA, exon-18-included and exon-18-skipped mRNAs are 488, 222 and 144 nt, respectively. Singly-spliced mRNAs migrate at 352 and 358 nt. Exons 17 and 19 are shown as light boxes, exon 18 as a dark box, and shortened introns as lines.

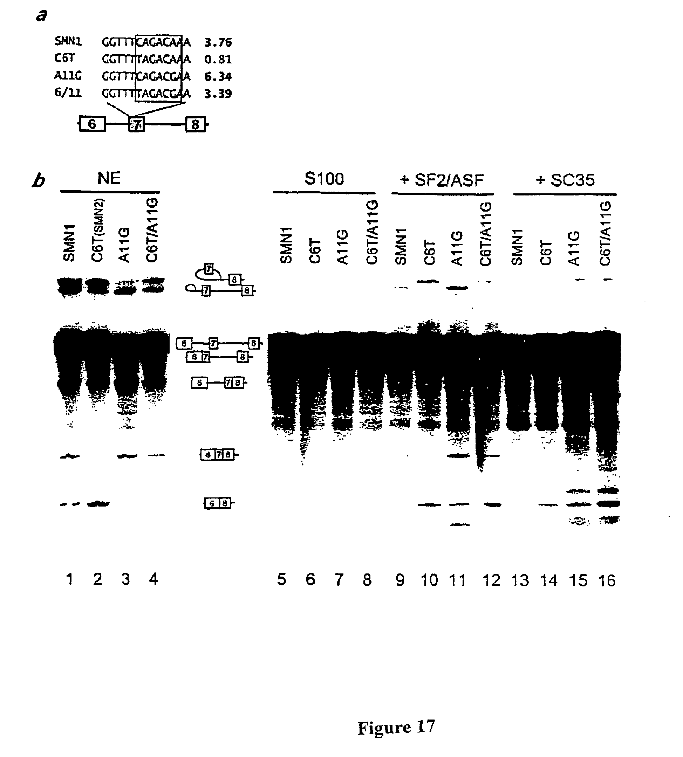

**Figure 17** illustrates that SF2/ASF promotes *SMN1* exon 7 inclusion *in vitro.* a.) *SMN* minigenes used for *in vitro* transcription and splicing. The relevant portion of *SMN1* exon 7 is shown above the diagram, starting with position +1 of each sequence. The calculated SF2/ASF motif scores corresponding to the highlighted heptamers are indicated for each minigene (high scores in black; sub-threshold score in gray). The minigenes are derivatives of those used in transfections, with smaller intron 6 and exon 8 to increase RNA stability and transcription and splicing efficiencies. b.) *In vitro* splicing of *SMN* minigenes reproduces the *in vivo* phenotype, and stimulation of exon 7 inclusion by SF2/ASF requires an SF2/ASF high-score motif The *SMN1*-derived pre-mRNAs corresponding to the wild type, or containing point mutations at position +6 (C6T, corresponding to *SMN2*), +11 (A11G), or both (C6T/A11G), were incubated for 4 hours under splicing conditions in HeLa nuclear extract (lanes 1-4), S100 extract alone (lanes 5-8), or S 100 extract complemented with 4 pmol of recombinant human SF2/ASF (lanes 9-12) or SC35 (lanes 13-16). The pre-mRNAs, intermediates and mature mRNAs are indicated schematically; flanking exons 6 and 8 are shown as open boxes, exon 7 as a gray box, and introns as lines. The sizes of pre-mRNA, exon-7-included and exon-7-skipped mRNAs are 910, 266 and 212 nt, respectively. Singly-spliced mRNAs migrate at 466 and 710 nt. The bands above the pre-mRNAs are the lariat intermediates. The structures of the additional bands seen only in the presence of SC35 have not been determined.

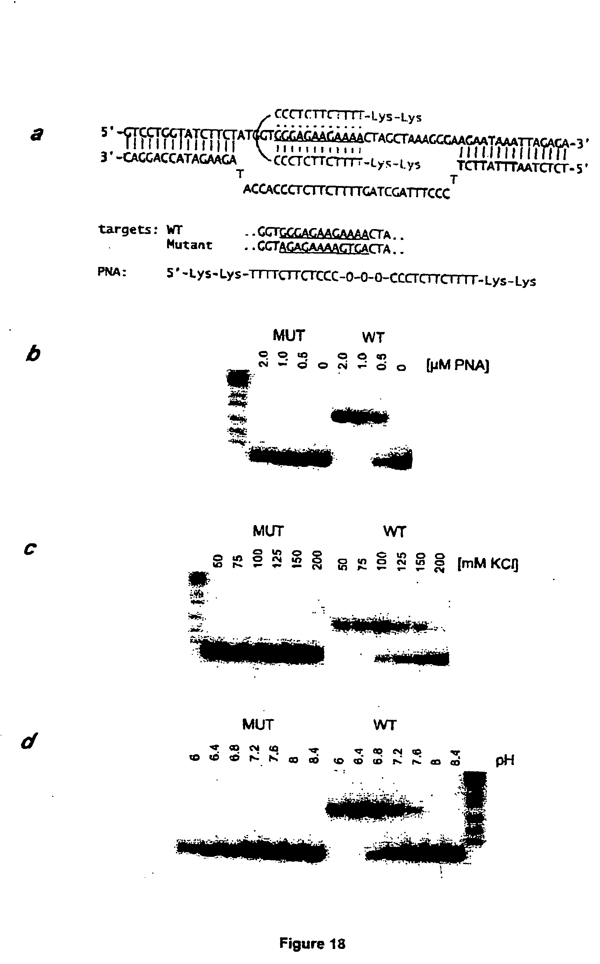

**Figure 18** illustrates specific targeting of double-stranded DNA by bis-PNA in vitro.
   a. Schematic representation of the bis-PNA bound to its dsDNA target. The vertical lines represent Watson-Crick base pairing, and the dots represent Hoogsteen base pairing. The PNA and wild-type and mutant target sequences are shown. The three Os denote three ethylene glycol linker residues.
   b. Electrophoretic mobility-shift assay, using a radiolabeled dsDNA target probe and unlabled PNA. Binding to the wild-type sequence is PNA-dose-dependent. No binding to the mutant sequence is observed, demonstrating the specificity.
   c. Electrophoretic mobility-shift assay showing the salt-dependence of binding.
   d. Electrophoretic mobility-shift assay showing the pH dependence of binding.
      The dsDNA target is from the human γ-globin promoter region, and binding of a similar bis-PNA-containing pseudoisocytosine instead of cytosine on the Hoogsteen strand-to the wild-type sequence was described in Wang et al. (1999) Nucleic Acids Res. 27:2806-2813. Modified cytosine is desirable for optimal binding at physiological pH.

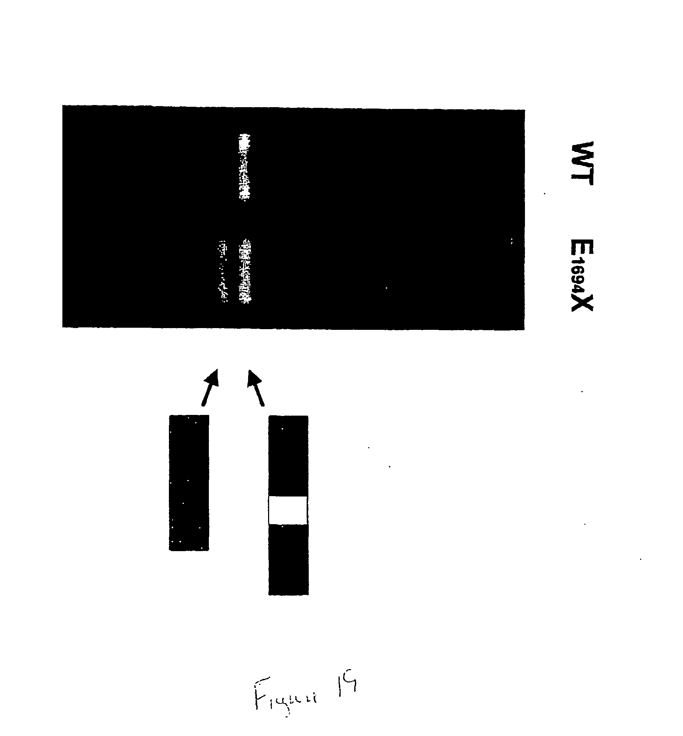

**Figure 19** illustrates expression of *BRCA1* in lymphoblast cell lines. Endogenous *BRCA1* mRNA was analyzed by RT-PCR with primers specific for exons 17 and 19. In the wild-type cell line only full-length mRNA with exon 18 included is detected. In the heterozygous mutant cell line, equal levels of exon 18 inclusion (from the wild-type allele) and skipping (from the mutant allele) are detected.

### DETAILED DESCRIPTION

The present invention provides chimeric molecules that include a base-pairing segment that binds specifically to a single-stranded nucleic acid molecule, and a moiety that modulates gene expression.

The base-pairing segment comprises purine and/or pyrimidine bases. The bases can be any naturally-occurring or modified purines and pyrimidines. Typically, the bases of the present invention are adenine, guanine, cytosine, thymidine and uracil.

These bases bind specifically to the bases of a target nucleic acid molecule according to the Watson-Crick rules of base-pairing. As a consequence of the precise nature of this binding, the base-pairing segment can be designed to anneal with any predetermined sequence of a nucleic acid molecule.

The bases can be modified, for example, by the addition of substituents at one or more positions on the pyrimidines and purines. The addition of substituents may or may not saturate any of the double bonds of the pyrimidines and purines. Examples of substituents include alkyl groups, nitro groups, halogens and hydrogens. The alkyl groups can be of any length, preferably from one to six carbons. The alkyl groups can be saturated or unsaturated; and can be straight-chained, branched or cyclic. The halogens can be any of the halogens including, bromine, iodine, fluorine or chlorine.

Further modifications of the bases can be the interchanging and/or substitution of the atoms in the bases. For example, the positions of a nitrogen atom and a carbon atom in the bases can be interchanged. Alternatively, a nitrogen atom can be substituted for a carbon atom; an oxygen atom can be substituted for a sulfur atom; or a nitrogen atom can be substituted for an oxygen atom.

Another modification of the bases can be the fusing of an additional ring to the bases, such as an additional five or six membered ring. The fused ring can carry various further groups.

Specific examples of modified bases include 2,6-diaminopurine, 2-aminopurine, pseudoisocytosine, E-base, thiouracil, ribothymidine, dihydrouridine, pseudouridine, 4-thiouridine, 3-methlycytidine, 5-methylcytidine, inosine, N⁶-methyladenosine, N⁶ -isopentenyladenosine, 7-methylguanosine, queuosine, wyosine, etheno-adenine, etheno-cytosine, 5-methylcytosine, bromothymine, azaadenine, azaguanine, 2'-fluoro-uridine and 2'-fluoro-cytidine.

The bases are attached to a molecular backbone. The backbone comprises sugar or non-sugar units. The units are joined in any manner known in the art.

In one embodiment, the units are joined by linking groups. Some examples of linking groups include phosphate, thiophosphate, dithiophosphate, methylphosphate, amidate, phosphorothioate, methylphosphonate, phosphorodithioate and phosphorodiamidate groups.

Alternatively, the units can be directly joined together. An example of a direct bond is the amide bond of, for example, a peptide.

The sugar backbone can comprise any naturally-occurring sugar. Examples of naturally-occurring sugars include ribose and deoxyribose, for example 2-deoxyribose.

A disadvantage of a base-pairing segment having naturally-occurring sugar units as the backbone is the possibility of cleavage by nucleases. Cleavage of the base-pairing segment can occur when the segment is in a single-stranded state, or upon specifically binding to a nucleic acid molecule.

Accordingly, it is preferable that the sugar units in the backbone are modified so that the modified sugar backbone is resistant to cleavage. The sugars of the backbone can be modified in any manner that achieves the desired cleavage resistance. Examples of modified sugars include 2'-O-alkyl ribose, such as 2'-O-methyl ribose and 2'-O-allyl ribose. Preferably, the sugar units are joined by phosphate linkers. The sugar units may be linked to each other by 3'-5', 3'-3' or 5'-5' linkages. Additionally, 2'-5' linkages are also possible if the 2' OH is not otherwise modified.

The non-sugar backbone can comprise any non-sugar molecule to which bases can be attached. Non-sugar backbones are known in the art.

In one embodiment, the non-sugar backbone comprises morpholine rings (tetrahydro-1,4-oxazine). (Loudon, G.M., Organic Chemistry, page 1178.) The resulting base-pairing segment is known as a morpholino oligo. (Summerton et al., Antisense Nucleic Acid Drug Dev. 7:187-195 (1997).) The morpholine rings are preferably joined by non-ionic phosphorodiamidate groups. Modified morpholines known in the art can also be used in the present invention. An example of a portion of a morpholino oligo is shown below, wherein "B" represents a base as described above.

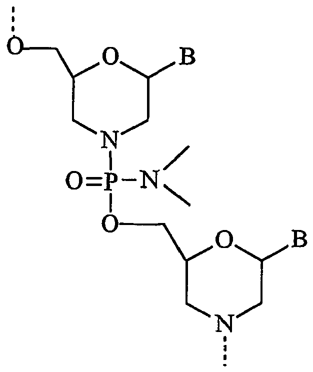

In another embodiment, the non-sugar backbone comprises modified, or unmodified, amino acid units linked by, for example, amide bonds. The amino acids can be any amino acid, including natural or non-natural amino acids, and are preferably alpha amino acids. The amino acids can be identical or different from one another. Examples of suitable amino acids include amino alkyl-amino acids, such as (2-aminoethyl)-amino acid.

Bases are attached to the amino acid backbone by molecular linkages. Examples of linkages are methylene carbonyl, ethylene carbonyl and ethyl linkages. The resulting pseudopeptide is known as a peptide nucleic acid (PNA). (Nielsen et al., Peptide Nucleic Acids-Protocols and Applications, Horizon Scientific Press, pages 1-19; Nielsen et al., Science 254: 1497-1500.)

An example of a PNA comprises units of N-(2-aminoethyl)-glycine. (See Figure 5.) Further examples of PNAs include cyclohexyl PNA, retro-inverso, phosphone, propionyl and aminoproline PNA. (Nielsen et al., Peptide Nucleic Acids-Protocols and Applications, Horizon Scientific Press, page 7.)

PNAs can be chemically synthesized by methods known in the art, e.g. by modified Fmoc or tBoc peptide synthesis protocols. PNAs have many desirable properties, including high melting temperatures (Tm), high base-pairing specificity with nucleic acid molecules and an uncharged backbone. Additionally, a PNA does not confer RNase H sensitivity on the target RNA, and generally has good metabolic stability.

The length of the base-pairing segment is not critical, as long as the length is sufficient to hybridize specifically to the target nucleic acid. For example, the base-pairing segment can have from about six to about one hundred bases, more preferably from about eight to about fifty bases, and most preferably from about ten to about twenty bases.

Various factors can be considered when determining the length of the base-pairing segment, such as target specificity, binding stability, cellular transport and *in vivo* delivery. For example, a base-pairing segment should be long enough to stably anneal to a target nucleic acid. Also, the segment should be long enough to allow for target specificity since, for example, a short sequence has a higher probability of occurring elsewhere in the genome vis-à-vis a long sequence. However, a base-pairing segment should not be so long that it binds too tightly to the target nucleic acid thereby possibly inhibiting late steps of splicing, or mRNA transport through the nuclear pore, or cytoplasmic translation of the mRNA. In addition, an excessively long base-pairing segment may anneal to secondary targets with partial complementarity. A further consideration is that the length of a base-pairing segment may affect the efficiency of *in vivo* delivery.

The nucleic acid molecule to which the base-pairing segment anneals may be any nucleic acid molecule. For example, the nucleic acid can be any single-stranded nucleic acid, including single-stranded RNA and DNA.

In one embodiment, the modulation of gene expression pertains to the modulation of RNA splicing. The base-pairing segment is joined to a moiety that modulates splicing, to form the chimeric molecules of the present invention. The modulation can be up-regulation or down-regulation of splicing. More than one chimeric molecule can be used to modulate splicing.

The present invention is not limited by any particular mechanism of splicing. At the time of filing this application, the mechanism of splicing is not fully defined, and the mechanism followed in one context is not necessarily followed in another context.

In this embodiment, the nucleic acid to which the base-pairing segment anneals is a pre-mRNA transcript. The base-pairing segment of the chimeric molecule anneals to a complementary region on the pre-mRNA transcript so that the moiety is brought to a position where it can modulate splicing of the pre-mRNA transcript. The moiety modulates splicing by promoting spliceosome assembly in proximity to a target splice site. The target splice site is the site on the pre-mRNA transcript where splicing is to be modulated.

Preferably, the base-pairing segment anneals to the pre-mRNA transcript at a position where the moiety can modulate the splicing without hindering binding of essential splicing factors to the 5' and 3' splice sites, the branch site, or the exon borders. For example, this position on the pre-mRNA can be next to the target splice site itself or up to 300 residues downstream or upstream from the target splice site, preferably from about two to about fifty residues from the target splice site, more preferably from about ten to about twenty-five residues from the target splice site. The region on the pre-mRNA to which the base-pairing segment anneals can be an exon or an intron. In some cases, it would be preferable to have the base-pairing segment anneal to an intron since in such a manner the chimeric molecule would never be bound to the spliced mRNA.

The moiety of the chimeric molecule used to modulate pre-mRNA splicing can be any moiety that modulates pre-mRNA splicing. The moiety preferably comprises a protein domain involved in splicing activation, i.e., a splicing activation domain. Such domains are known in the art. In one example, the protein domain occurs naturally, such as in an SR protein. SR proteins are proteins that have a domain rich in serine-arginine dipeptides. Examples of naturally-occurring SR proteins include SF2/ASF, SC35, SRp40 and SRp55. Active fragments of these naturally-occurring protein domains can also be used as the moiety. Another example of a splicing activation domain comprises a sequence rich in arginine-glutamic acid dipeptides.

The domain involved in splicing activation can also be a synthetic sequence that has been designed to have a function that is similar to that of the naturally occurring protein domain. An example of a synthetic domain with a function similar to a naturally occurring protein domain comprises a sequence that is rich in arginine-serine dipeptides. At least one serine can be replaced with a glutamate or aspartate to mimic a constitutively phosphorylated domain. Another example of a synthetic domain, with function similar to that of a natural splicing activation domain, comprises a sequence that is rich in arginine-glutamic acid dipeptides.

Alternatively, the moiety can be synthetic, short polymers with alternating charge. Such polymers are called polyampholytes. (Hampton et al., Macromolecules 33: 7292-7299 (2000); Polymeric Materials Encyclopedia, Salamone, Ed., CRC Press (1996).) Preferably, these polymers contain monomers with dimensions similar to that of arginine and phosphoserine. Additionally, the spacing between the monomers is preferably similar to that of the spacing between arginine and phosphoserine.

The length of the domain involved in splicing activation can vary. For example the domain can include from about three to about two hundred amino acid residues, more preferably from about five to about one-hundred residues, and most preferably from about fifteen to about thirty residues.

Analogously, the number of dipeptide repeats in the domain can also vary. For example, the number of dipeptide repeats can be from about two to about one hundred repeats, more preferably from about five to about fifty repeats, even more preferably from about eight to about twenty-five repeats, and most preferably from about ten to about fifteen repeats.

There are several factors to be considered when determining the length of the splicing activation domain. For example, longer domains may be more potent; however, chimeric molecules produced for therapeutic intervention, in most cases, should be as small as possible.

In another embodiment, the moiety is a protein or a single-stranded or a double stranded nucleic acid molecule that includes a binding site for a splicing protein. The splicing protein that binds to this moiety is preferably a splicing protein that is endogenous to an organism, such as a SR protein. In another embodiment the splicing protein can be exogenous, including naturally-occurring and synthetic proteins. Some examples of splicing proteins are those containing the splicing activation domains described above.

In a preferred embodiment, the moiety that includes a splicing protein-binding site is an RNA segment. The end of the RNA segment that is not joined to the base-pairing segment, optionally, has adjoining non-RNA residues. These non-RNA residues protect the RNA from ribonucleases. A few examples of such non-RNA residues include amino acid residues; modified oligonucleotides, such as 2'-O methyl oligonucleotides; morpholino oligos and PNAs.

In another embodiment, the moiety is a modified RNA. The modified RNA can be any modified RNA that includes a binding site for a splicing protein. An example of such a modified RNA is 2'-O methyl RNA.

In another embodiment, the moiety is a small molecule that modulates splicing; or a small molecule that binds specifically to a splicing protein or splicing protein domain. For example, small molecules that bind specifically to a splicing protein, or splicing domain, can be obtained by screening chemical, combinatorial, phage display or RNA aptamer libraries. In one embodiment, the small molecule can be biotin. In this case, a splicing protein or splicing domain can be fused to avidin or streptavidin.

In one embodiment, the modulation of pre-mRNA splicing pertains to enhancing the inclusion of certain portions of the pre-mRNA transcript, i.e. a target exon, into the spliced mRNA. The use of the chimeric molecules of the present invention to promote exon inclusion has many applications.

For example, promotion of exon inclusion can be used to improve or restore correct RNA splicing for defective genes in which inappropriate exon skipping results from mutations. These mutations include missense, nonsense, synonymous and frameshift mutations; and small intra-exonic deletions and insertions.

For example, the chimeric molecules of the present invention can promote exon inclusion where an exonic splicing enhancer (ESE) is absent or has been wholly or partially inactivated by a mutation, or a single nucleotide polymorphism. ESEs are sequences which are present in either constitutive or alternative exons of certain genes, and are required for those exons to be spliced efficiently. It is believed that when a normal ESE is present, one or more SR proteins bind to the pre-mRNA transcript via the proteins' RNA-recognition motif(s). (See Figure 2.) Each SR protein recognizes a unique, albeit highly degenerate ESE sequence motif under splicing conditions. (See Figure 3.) The arginine-serine-rich domain of the SR protein serves to promote spliceosome assembly at the splice site(s) flanking an exon thereby enhancing inclusion of the ESE-containing exon in the spliced mRNA. If an ESE is absent or has been inactivated, binding of an SR protein may be precluded; and as a result, exon recognition is impaired.

In order to compensate for the absent or inactive ESE, the base-paring segment of the chimeric molecules of the present invention are designed so that they anneal to a target sequence on the pre-mRNA transcript by base-pairing. Once bound, the moiety of the chimeric molecule can promote spliceosome assembly at a target splice site flanking a particular exon, thereby promoting the inclusion of the exon.

For example, the defective splicing of a mutant *BRCA1* transcript can be corrected by the chimeric molecules of the present invention. An amber nonsense mutation (Glu1694Ter) involving a G to T transversion at position 6 of exon 18 of the breast cancer susceptibility gene *BRCA1* causes inappropriate skipping of the entire constitutive exon 18 *in vivo.* (Mazoyer et al., Am. J. Hum. Genet. 62:713-715 (1998).) This mutation was found in a family with eight cases of breast cancer or ovarian cancer. The identical mutation in genomic DNA was also reported five times in the 2000 BRCA1 Information Core Database. Skipping of exon 18 results in retention of the same reading frame and removal of 26 amino acids, disrupting the first BRCT domain of *BRCA1.*

In one example of the present invention, the chimeric molecule used to promote exon inclusion was a twelve-residue PNA joined to a twenty-two residue peptide. (See Figure 6.) The PNA bases were complementary to a segment of *BRCA1* exon 18, just downstream from the mutant site on the exon. The peptide portion of the chimeric molecule in this example included ten arginine-serine (RS) dipeptide repeats. The chimeric molecule effectively promoted exon 18 inclusion in the spliced mRNA.

Exon skipping can also result from mutations in introns, at or near splice sites, or from mutations that activate cryptic splice sites. The present invention includes promotion of exon inclusion in these situations. As stated above, the chimeric molecules can be used to promote spliceosome assembly at a target splice site on the pre-mRNA transcript.

The base-pairing segment does not have to anneal directly across a mutation. As stated above, the base-pairing segment is required only to anneal to a position on the pre-mRNA where it can promote spliceosome assembly at splice sites flanking a target exon. This position is not necessarily on a mutation.

There may be multiple alleles of a given gene with a certain mutation. Since it is not required that the base-pairing segment anneal directly across a mutation, a single chimeric molecule of the present invention can be used to correct exon skipping in all of the alleles that cause skipping of a particular exon.

In one embodiment, the chimeric molecules promote inclusion of an exon in a mRNA transcript where the inclusion does not occur naturally, or where the inclusion occurs only partially.

For example, splicing of exon 7 of the *SMN2* gene can be promoted by the chimeric molecules of the present invention. The *SMN2* gene is almost identical to the *SMN1* gene, except that splicing of the *SMN2* gene fails to efficiently include exon 7. (See Figure 1) The *SMN2* gene differs only in subtle ways from the *SMN1* gene, but only the latter is thought to be critical for viability and for proper motor neuron function in normal individuals.

In individuals with spinal muscular atrophy (SMA), however, both copies of the *SMN1* gene are missing or are grossly defective. The patients survive, albeit with SMA disease, because they have one or more copies of the *SMN2* gene. Splicing of the *SMN2* pre-mRNA yields mostly mRNA in which the penultimate exon (exon 7) is skipped. Messenger RNA which includes exon 7 is generated only at low levels.

It has been shown that exon 7 is predominantly skipped in *SMN2* pre-mRNA and included in *SMN1* pre-mRNA because of the presence of a cytosine at position +6 of exon 7 in the *SMN1* gene versus a thymine at the same position in the *SMN2* gene. The chimeric molecules of the present invention can be targeted so that *SMN2* exon 7 is included in the mRNA transcript. The cytosine and thymine at this position are part of synonymous codons, and hence *SMN2* mRNA containing exon 7 encodes fully functional survival-of-motor-neuron protein.

In another embodiment, the modulation of pre-mRNA splicing pertains to modulating alternative splicing. Alternative splicing includes any variations in the processing of pre-mRNA that allow more than one possible protein to be made from a single gene. For example, a pre-mRNA transcript can be spliced in various ways so that the final mRNA can appear in multiple isoforms.

The chimeric molecules of the present invention can promote the formation of a particular isoform vis-à-vis a different isoform. For example, the chimeric molecules can be used to enhance a particular alternative splicing pathway vis-à-vis a different splicing pathway. As described above, the chimeric molecule anneals to a position on the pre-mRNA transcript whereby the molecule can promote formation of a spliceosome assembly in proximity to a target splice site. The chimeric molecules can thus force the inclusion of specific exons in the mRNA transcript to result in the ectopic expression of particular isoforms.

Through modulation of alternative splicing, the chimeric molecule can also decrease the expression of a gene, or one or more of its isoforms. For example, one of the alternative exons may contain an in-frame nonsense codon, resulting in degradation of the spliced mRNA by nonsense-mediated decay. In another example, a non-functional truncated peptide is encoded when an alternative exon is included. Targeting the chimeric molecule to promote inclusion of such exons would downregulate the expression of a particular gene or reduce the activity of the protein encoded. Genes to which such downregulation can be targeted include, for example, an oncogene or viral gene.

The chimeric molecule can also be used to improve gene expression. For example, in some cases of gene expression splicing of a particular intron is a rate-limiting step. Unspliced or partially spliced transcripts usually accumulate in the nucleus and are not accessible to the protein synthesis machinery. The chimeric molecule can be targeted so as to increase the rate of splicing of the rate-limiting intron from the pre-mRNA transcript. In other cases of gene expression, there is an intron that normally remains largely unspliced. The chimeric molecule can force the splicing of such an intron. In both these cases the use of the chimeric molecule can result in an increase of fully spliced mRNA that is available for transport to the cytoplasm and for translation, thus resulting in increased protein production.

The chimeric molecules can promote pre-mRNA splicing that does not occur naturally, or that occurs only partially. As described above, a chimeric molecule is targeted to any position on the pre-mRNA transcript where promotion of spliceosome assembly is desired.

For example, splicing can be forced in a virus or a retrovirus. In particular, viruses, such as the HIV retrovirus, have evolved signals and mechanisms to allow transport of unspliced or partially spliced mRNAs in addition to fully spliced mRNAs. The viral life cycle requires proteins encoded by all of these RNAs. Thus, increasing the removal of some or all of the viral introns by splicing (oversplicing) would be detrimental to the virus. The chimeric molecules can be targeted to one or more viral exons to promote such splicing.

The modulation of pre-mRNA splicing may also pertain to correcting defective splicing. Defective splicing is splicing of a pre-mRNA transcript that results in a defective protein product. Typically, the splicing of the transcript is defective due to small defects, i. e. mutations, in the genetic material which are carried forward to the pre-mRNA transcript. The defective splicing can result in formation of a spliced mRNA transcript which contains an exon which is larger or smaller than the corresponding normal exon; formation of a completely new exon not found in the normal transcript; elimination of an exon needed to express a normal protein product; or a fusion of an exon of one gene with the exon of another gene. These defects result in defective protein products.

The modulation of gene expression may also pertain to the modulation of translation. The modulation can be up-regulation or down-regulation of translation. The base-pairing segment is joined to a moiety that modulates translation, to form the chimeric molecules. The nucleic acid molecule to which the base-pairing segment anneals is an mRNA transcript. More than one chimeric molecule can be used to modulate translation. The present invention is not limited by any particular mechanism of translation. Preferably, PNA-peptides can be used to anneal to the mRNA.

More specifically, the base-pairing segment of the chimeric molecule anneals to a complementary region on the mRNA transcript so that the moiety is brought to a position where it can modulate translation of the mRNA transcript. Translation requires the presence of various factors, co-factors and building blocks, besides the mRNA template, including ribosomes; amino-acylated tRNAs; initiation, elongation and release protein factors; GTP; ATP; etc. The moiety of the chimeric molecule recruits one or more of these components to the mRNA to be translated.

The moiety can include, for example, a peptide sequence of the rotavirus nonstructural protein NSP3. In particular, the peptide sequence can be (MYSLQNVISQQQSQIADLQNYCNKLEVDLQNKISSLVSSVEWYLKSMELPDE IKTDIEQQLNSIDVINPINAIDDFESLIRNIILDYDRIFLMFKGLMRQCNYEYTYE) (SEQ. ID. NO. : 1). (Piron et al., Journal of Virology 73: 5411-5421 (1999); Vende et al., Journal of Virology 74 : 7064-7071 (2000).) The action of this peptide sequence includes the recruitment of eukaryotic initiation factor 4GI (eIF4GI).

Alternatively, the moiety can include the N-terminal domain of the influenza virus NS I protein, in particular the first one hundred thirteen amino acids of the N-terminal domain. (Aragon et al., MCB, 20: 6259-6268 (2000).) The action of this domain also includes the recruitment of eukaryotic initiation factor 4GI (eIF4GI).

Alternatively, the moiety can include domains of poly (A)-binding protein (PAB). In particular, the RNA-recognition motif (RRM) domains 1 and 2, i. e. amino acids 1-182 of the PAB protein. A binding site for eIF-4G lies in RRMs I and 2. EIF- 4G forms part of a cap-binding complex with eIF-4E. (Gray et al., EMBO, 19: 47234733 (2000).)

The modulation of gene expression may also pertain to the modulation of transcription. The base-pairing segment is joined to a moiety that modulates transcription to form the chimeric molecules. The moiety can be a peptide which comprises up to about one hundred amino acid residues. Modulation can be upregulation or down-regulation of transcription. More than one chimeric molecule can be used to modulate transcription.

The target nucleic acid to which the base-pairing segment anneals is a doublestranded nucleic acid molecule. The nucleic acid can be any double-stranded nucleic acid molecule, including double-stranded DNA, double-stranded RNA and mixed duplexes between DNA and RNA.

Preferably, the chimeric molecules are targeted to double-stranded DNA. Any position on the DNA that allows the moiety to recruit various transcription factors to, for example, promoter or enhancer elements on the DNA may be targeted. The chimeric molecules bind to the double-stranded DNA in any manner in which the chimeric molecules can base-pair to the double-stranded DNA.

For example, a base-paring segment can bind to double-stranded DNA by strand displacement. The base-pairing segment can bind to DNA in either a parallel or an antiparallel orientation.

A strand displacement complex is formed by a chimeric molecule that has a homopyrimidine base-pairing segment and a second molecule. A homopyrimidine base-pairing segment has several pyrimidines in a row. For example, the homopyrimidine base-pairing segment can have five to twenty pyrimidines in a row, more preferably ten to fifteen pyrimidines in a row. The second molecule can be a PNA, modified oligo or another chimeric molecule.

The base-pairing segment of the chimeric molecule binds by Watson-Crick base-pairing to a target segment of a DNA strand. The second molecule forms Hoogsteen hydrogen bonds with the same DNA strand. Thus, a clamp is formed with two molecules binding one DNA strand. The DNA stretch complementary to the target DNA is displaced and remains single stranded. The resultant complex is termed, a "triplex invasion."

Preferably, the base-pairing segment is a PNA. Accordingly, the "triplex invasion" can be represented as PNA·DNA-PNA/DNA, where "·" represents Hoogsteen hydrogen bonds and "-" represents Watson-Crick base-pairing. In one embodiment, two PNA strands may be covalently connected by a flexible linker and are thus termed bis-PNA.

Alternatively, a strand displacement complex can be formed by a chimeric molecule comprising a homopurine base-pairing segment. A homopurine base-pairing segment has several purines in a row. For example, the homopurine can have five to twenty purines in a row, more preferably ten to fifteen purines in a row. The base-pairing segment of a single chimeric molecule binds the target DNA via Watson-Crick base-pairing. The DNA stretch complementary to the target DNA is displaced and remains single stranded. The resultant complex is termed, a "duplex invasion."

Preferably, the base-pairing segment is a PNA. Accordingly, the "duplex invasion" can be represented as PNA-DNA/DNA, where "-" represents Watson-Crick base-pairing.

Alternatively, a strand displacement complex can be formed by a chimeric molecule and a second molecule, both of which comprise pseudo-complementary base-pairing segments. The base-pairing segments are termed pseudo-complementary because adenine and thymine bases are replaced with diaminopurine and thiouracil bases, respectively. The formation of base-pairing segment duplexes is prevented by the diaminopurine and thiouracil bases. The second molecule can be a PNA, modified oligo or another chimeric molecule.

These base-pairing segments achieve strand displacement by the formation of two duplexes via Watson-Crick base-pairing. The resultant complex is termed "double-duplex invasion."

Preferably, the base-pairing segment is a PNA. Accordingly the "double-duplex invasion" can be represented as PNA-DNA/PNA-DNA where "-" represents Watson-Crick base-pairing.

The moiety that modulates transcription can be any transcription activation domain. The length of this domain is preferably the minimum length that has the desired activity. Multiple domains provide increased activity. For example, such a domain can have up to one hundred residues, preferably up to fifty residues and most preferably up to thirty residues. An example of such a domain is AH (PEFPGEELQELQELQALLQQ) (SEQ. ID. NO.:2). (Giniger et al., Nature (London) 330, 670-2 (1987.) Another example is human oct-2 glutamine-rich peptide, Q18III. This domain is eighteen amino acids long. Preferably, three tandem copies are used to give strong activity in a protein context. (Tanaka and Herr, Mol Cell Biol 14: 6056-67 (1994).) Another example of a transcription activation domain is NF-kappa B RelA (p65) subunit acidic activation module. This domain is eleven amino acids long. Preferably, two tandem copies are used to give strong activity. (Blair et al, Mol Cell Biol 14: 7226-34 (1994).) Other examples are homopolymeric activation modules. These activation modules contain ten to thirty glutamines, or about ten prolines. (Gerber et al, Science 263: 808-811 (1994).) Another example is a VP16 activation domain derived peptide. This domain comprises eleven amino acids (DALDDFDLDML). (SEQ. ID. NO.:3). Other peptides derived from this natural sequence can be used which are fifteen to twenty amino acids in length and have specific arrays of aspartate and leucine residues. (Seipel et al, Biol. Chem. Hoppe Seyler 375: 463-70 (1994).)

To achieve modulation of gene expression, a gene expression system is contacted with the chimeric molecules. The gene expression system refers to any system in which genes may be expressed. The gene expression system may be *in vitro, ex vivo* or *in vivo. In vitro* systems typically include cultured samples and cell-free systems. *Ex vivo* systems typically include cells or organs removed from a living animal. *In vivo* systems include living animals. Thus, the gene expression system includes, but is not limited to, any cell, tissue, organ, whole organism or *in vitro* system that expresses the gene while in contact with the chimeric molecules.

The chimeric molecules can be modified to optimize their use for various applications. In particular, these methods include modifications to improve delivery, cellular uptake, intracellular localization, pharmacokinetics, etc.

One manner in which the chimeric molecules can be modified is by the addition of specific signal sequences. The signal sequences may be incorporated into the chimeric molecules at any point during synthesis.

For example, nuclear retention signals (NRS) can be incorporated into the chimeric molecules. In particular, the effectiveness of the chimeric molecules in modulating pre-mRNA splicing can be improved if, once the molecules are imported to the nucleus, they are efficiently retained there. Nuclear retention can preclude, for example, the possibility of toxicity due to unwanted inhibition of cytoplasmic translation of mature mRNA. However, the off rates of chimeric molecules bound to the mRNA transcript need to be considered. For example, the stable hybridization of chimeric molecules targeted to exon 7 of the SMN pre-mRNA transcript coupled with dominant retention signals, may preclude mRNA export, and hence preclude the synthesis of SMN protein. (In this case, it is preferred that the chimeric molecules target the intronic regions of the SMN pre-mRNA transcript so that the chimeric molecules would not associate with the mature mRNA.) Examples of NRSs include the hnRNP C nuclear retention signal (Nakielny et al., J. Cell Biol. 134(6):1365-73 (1996).)

Additionally, signal sequences which enhance transport across cell membranes may be incorporated, such as polylysine, poly(E-K), and nuclear localization signals.

Also signal sequences that promote transport across the brain-blood barrier (BBB) can be incorporated. Transport across the BBB can be either by diffusion or by saturable receptor systems. Examples of signals that would promote transport across the BBB is the Dowdy Tat peptide, and peptide sequences that are part of MIF-1, leptin, interleukin-1, and epidermal growth factor. (Kastin et al., Brain Res. 848 (1-2):96-100 (1999).)

Also signal sequences that promote transport across the placental barrier can be incorporated. (Chandorkar et al., Adv. Drug Deliv. Rev. 14;38(1):59-67 (1999); Simister et al., Eur. J. Immunol. 26(7):1527-31 (1996).)

Additionally, signal sequences can be included if it is desired to target the chimeric molecule to different cell types or different parts of a cell. In an example of an *in vivo* application of this invention, the chimeric molecules are administered to SMA patients. In this case, the chimeric molecule can include a small peptide ligand that is specific for a neuromuscular junction receptor.

Additionally, cellular uptake can be enhanced by the addition of a protein transduction domain on either side of the moiety. The transduction domain can be an amphipathic helix with multiple basic amino acids that may interact with the anionic face of the plasma membrane. Preferred protein transduction domains include residues derived from the N-terminus of HIV-TAT protein (e.g., YARAAARQARA (SEQ ID NO:4) and YGRKKRRQRRR (SEQ ID NO.: 5)). Additionally, peptides derived from Drosophila Antennapedia are also effective. All these domains facilitate bi-directional passage across the plasma membrane of relatively large or very large molecules that are normally not internalized. A preferred chimeric molecule, which modulates splicing, is a PNA-peptide with the shortest arginine-serine domain determined to be active with the TAT peptide juxtaposed to either the N-terminal or C-terminal end of the domain.

Additionally, transport across cell membranes can be enhanced by combining the chimeric molecule with a. carrier. Some examples of suitable carriers include cholesterol and cholesterol derivatives; liposomes; protamine; lipid anchored polyethylene glycol; phosphatides, such as dioleoxyphosphatidylethanolamine, phosphatidyl choline, phosphatidylglycerol; α-tocopherol; cyclosporin; etc. In many cases, the chimeric molecules can be mixed with the carrier to form a dispersed composition and used as the dispersed composition.

The chimeric molecule can be administered to mammals in any manner that will allow the chimeric molecules to modulate gene expression. Mammals include, for example, humans; pet animals, such as dogs and cats; laboratory animals, such as rats and mice; and farm animals, such as horses and cows. Additionally, mammals, for the purposes of this application, include embryos, fetuses, infants, children and adults. Examples of the administration of the chimeric molecules include various specific or systemic administrations, e.g., injections of the chimeric molecules.

For example, the appropriate chimeric molecules can be delivered to SMA patients in any manner that allows for enhancement of the incorporation of exon 7 of the *SMN2* gene. The chimeric molecules are preferably delivered *in utero* or at an appropriate time after birth. In the mouse model, an appropriate time is forty-eight hours after birth. An appropriate time after birth for humans is the time that corresponds to forty-eight hours in the mouse model. The administration of the chimeric molecules at a significant time after birth can prevent further degeneration of motor neurons and/or partially reverse the course of a disease after its onset. A significant time after birth can prevent further degeneration of motor neurons and/or partially reverse the course of a disease after its onset. A significant time after birth can be up to the appearance of motor neuron degenerative symptoms, or after the onset of the disease. Also the chimeric molecules can be administered throughout the lifetime of a patient.

Further disclosed herein is a method of making the chimeric molecules. The chimeric molecules are formed by joining the base-pairing segment and the moiety. The base-pairing segment can be joined to the moiety in any manner that will allow the base-pairing segment to be covalently bound to the moiety.

For example, a peptide moiety and a base-pairing segment can be separately synthesized and then chemically conjugated to one another. Several peptide moieties can be conjugated to a single base-pairing segment. Alternatively, several basepairing segments can be conjugated to a single moiety.

The structure of a PNA-peptide conjugate to be used in the present invention can be C-peptide-N-5'-PNA-3' ; C-peptide-N-3'-PNA-5' ; N-peptide-C-5'-PNA-3' ; N-peptide-C-3'-PNA-5' ; 5'-PNA-3'-C-peptide-N; 5'-PNA-3'-N-peptide-C ; 3'-PNAS'-C-peptide-N or 3'-PNA-5'-N-peptide-C.

A PNA may be conjugated to a peptide by methods known in the art. See, for example, Tung et al., Bioconjug Chem. 2: 464-5; Bongartz et al. Nucleic Acid Res. 22: 4681-8; Reed et al., Bioconjug Chem. 6: 101-108; and de La Torre et al. Bioconjug C'hea. 10: 1005-1012.

A PNA and a peptide moiety can be incorporated sequentially during synthesis in a single automated machine, thereby obviating postsynthesis conjugation steps. The single automated machine can be a peptide synthesizer or certain modified oligonucleotide synthesizers. Either the moiety or the PNA can be synthesized first. Peptides are synthesized from C- to N-terminus, and PNA from 3' to 5'. Thus, chimeric molecules can be made in a single step as N-peptide-C-5'-PNA-3' or 5'-PNA-3'-N-peptide-C.

The chimeric molecule can optionally include a spacer sequence between the base-pairing segment and the moiety. The spacer sequence advantageously provides conformational flexibility to the molecule. The spacer can include any series of atoms or molecules.

For example, the units of the spacer sequence can be made of amino acid residues. The residues in the spacer are either the same or any combination of amino acid residues. Preferably, the residues have an inert character. In a preferred embodiment the amino acid residues are one or more glycine residues.

Additionally, the units of the spacer can be made of inert alkyl groups, e.g., methylene groups.

In another embodiment; one or more hydrophilic linkers can be introduced into the spacer during chemical synthesis. An example of a hydrophilic linker monomer is amino-3,6-dioxaoctanoic acid.

The length of the spacer sequence can vary. The spacer typically includes from about one to about one hundred units; more preferably from about two to about fifty units; most preferably from about five to about fifty units.

A PNA has the advantage that it can be coupled to a peptide moiety via automated synthesis. Other base-pairing segments can be covalently joined by a chemical conjugation reaction. To facilitate the joining of the base-pairing segment and the moiety, the base-pairing segment can include a nucleotide with a reactive functional group. The reactive functional group can be any functional group that facilitates coupling. Examples of reactive functional groups include reactive amino, sulfhydryl and carboxyl groups. An example of a reactive amino group is N-hexylamino.

For example, a derivatized nucleotide with an alkyl amino, e. g. an N-hexylamino group, can be incorporated into the base-pairing segment. In this embodiment, the peptide moiety includes, for example, an N-terminal cysteine.

Additionally, or alternatively, reactive groups can be included on the peptide moiety.

Alternatively, the base-pairing segment and the peptide moiety can be joined by means of a bifunctional crosslinker. The bifunctional crosslinker can be a heterobifunctional crosslinker, such as N- [y-maleimidobutyryloxy] sulfosuccinimide ester. This crosslinker provides a 6.8 A spacer (J. Immunol. Methods, 1988 Aug 9; 112 (1) : 77-83). Additionally, homo-bifunctional crosslinkers can be used.

The chimeric molecule may have a linear structure. In an embodiment of the invention the chimeric molecule has a branched structure. In a branched structure, the moiety is attached to an internal residue of the base-pairing segment; or the basepairing segment is attached to an internal residue of the moiety.

Further disclosed herein are methods for modulating expression of a nucleic acid molecule. The methods comprise contacting an appropriate nucleic acid molecule with any of the chimeric molecules described above. The chimeric molecules bind to the nucleic acid molecule at any location that allows the moiety to modulate expression.

In one example, the invention relates to a method for modulating splicing and/or translation. The method comprises contacting a single-stranded nucleic acid molecule with any of the chimeric molecules described above that comprises: a) a base-pairing segment that specifically binds to a portion of a single-stranded nucleic acid molecule; and b) a moiety that modulates splicing and translation. The single-stranded nucleic acid molecule may, for example, be a pre-mRNA transcript.

The chimeric molecule binds to the single-stranded nucleic acid molecule, e.g., a pre-mRNA transcript, at any location that allows the moiety to modulate splicing and translation. For example, the chimeric molecule binds to the single-stranded nucleic acid molecule at about 0 to about 300 residues from a splice site on the nucleic acid molecule. The binding may, for example, occur in either an intron or an exon.

The method may, for example, result in modulation of the rate of splicing, or in modulation of alternative splicing. Modulation of alternative splicing may, for example, result in an increase or in a decrease of the expression of a gene. Decreasing the expression of a gene is advantageous, for example, in the case of an oncogene or a viral gene. Alternatively, modulation of splicing promotes inclusion of a target exon in a mRNA transcript. Such inclusion is desirable when, for example, an exon fails to be spliced because an exonic splicing enhancer of the exon is absent or inactive. The exonic splicing enhancer may, for example, be absent or inactive due to a nonsense mutation, missense mutation, synonymous mutation, frameshift mutation, intra-exonic deletion, intra-exonic insertion or single-nucleotide polymorphism.

The target exon may, for example, be an exon of the SMN2 gene, such as exon 7 of the SMN2 gene. Delivery of exon 7 of the SMN2 gene is important, for example, in the case of patients with spinal muscular atrophy. Exon 7 may, for example, be introduced into a gene either *in utero* or *ex utero.*

In a preferred embodiment of the method described above, the invention relates to a method to correct defective splicing of a pre-mRNA transcript during pre-mRNA splicing. The method comprises contacting the pre-mRNA transcript with any of the chimeric molecules described above that comprise: a) a base-pairing segment that specifically binds to the pre-mRNA transcript; and b) a moiety that modulates splicing. The binding of the base-pairing segment allows the moiety to correct defective splicing.

In another embodiment, the invention relates to a method for modulating transcription. The method comprises contacting a double-stranded nucleic acid molecule with any of the chimeric molecules described above that comprise: a) a base-pairing segment that specifically binds to a portion of the double-stranded nucleic acid molecule; and b) a moiety that modulates transcription. The chimeric molecules bind to the double-stranded nucleic acid molecule at any location that allows the peptide to modulate transcription. The moiety is preferably a peptide which comprises from about two to about one hundred amino acid residues.

In a final embodiment, the invention relates to a method of making any of the chimeric molecules described above. The method comprises covalently bonding a base-pairing segment that binds specifically to a nucleic acid molecule, and a moiety that modulates gene expression

### EXAMPLES

The following examples are intended to show the practice of the invention and are not intended to restrict the scope of the present invention.

### Example 1. SR Protein Motifs

A functional SELEX strategy coupled with the S 100 complementation assay was developed to define the role of SR proteins in constitutive splicing. By means of this strategy sequence motifs that act as functional enhancers in the presence of the cognate recombinant SR protein were defined. Figure 3 shows the motifs that were found for four SR proteins, displaying each nucleotide with a size proportional to its frequency at that position of the consensus. Each consensus was derived from an alignment of ~30 functional sequences selected by splicing in the presence of a single SR protein. The motifs are highly degenerate, probably reflecting evolutionary constraints on the presence of exonic splicing signals within a vast set of unrelated protein-coding segments. The degeneracy is also consistent with the RNA-binding properties of SR proteins, which exhibit significant sequence preferences, but nevertheless can bind reasonably tightly to very diverse RNA sequences. Thus, a relatively small number of SR proteins can mediate enhancement via elements present in an extremely diverse set of exons. Additional diversity and specificity are probably achieved through other factors that act as activators or co-activators of SR proteins, such as SRm160/300 or the Tra2 proteins.

Statistical methods were used to evaluate the occurrence of the enhancer motifs, identified by SELEX, in natural sequences. Using nucleotide-frequency scoring matrices, the motifs for four SR proteins (SF2/ASF, SC35, SRp40 and SRp55) were found to be more prevalent in exons than in introns, and tend to cluster in exonic regions corresponding to known natural enhancers. Each type of motif appears to be necessary for enhancement when the cognate SR protein is the sole one present in the S100 complementation assay. However, the presence of a motif is not sufficient for activity, as context can be extremely important.

### Example 2. Mechanism of Exon Skipping in the BRCA1 gene

The recently derived SF2/ASF, SC35, SRp40, and SRp55 motif-scoring matrices were used to analyze the wild-type and a particular familial mutation in exon 18 of *BRCA1.* Multiple high-score motifs for each type of ESE are distributed throughout this exon (Fig. 13). The mutation at position 6 specifically disrupts the first of three high-score SF2/ASF motifs. To study the mechanism of exon skipping, wild-type and mutant minigenes were constructed. These minigenes include exons 17 through 19 and shortened versions ofintrons 17 and 18.

Radiolabeled transcripts from these minigenes were spliced *in vitro* (Fig. 14). The two pre-mRNAs were spliced in strikingly different ways: with wild-type pre-mRNA (WT), exon 18 was efficiently included (lane 1), whereas with mutant pre-mRNA (NT), exon 18 was predominantly skipped (lane 2). Figure 4 shows the time course results of the *in vitro* splicing assay.

Although the extent of exon inclusion and skipping varied with different extract preparations or buffer conditions, the ratio of exon skipping over inclusion was reproducibly greater with the mutant pre-mRNA. The overall recovery of labeled RNA was not significantly affected by the mutation (Fig. 14), making differential mRNA stability an unlikely explanation for the different splicing patterns observed. This result is consistent with the SF2/ASF high-score motif distribution, strongly suggesting that the nonsense mutation disrupted an ESE.

There is no *a priori* reason why ESE inactivation should result preferentially from in-frame nonsense mutations, as opposed to other types of base substitution. To examine the requirement for a nonsense mutation, two additional *BRCA1* minigene transcripts were designed (Fig. 15*a*). One of the mutant pre-mRNAs, ML, has a G to A transition at the same position as the original mutation, and is a missense mutation that also eliminates the high-score SF2/ASF motif. The other mutant pre-mRNA, NH, has an amber nonsense mutation in the following codon, but maintains a high-score SF2/ASF motif. Splicing of the wild-type and the three mutant transcripts was compared *in vitro,* and quantitation of the relative extent of exon 18 inclusion is shown (Fig. 15*b*). Splicing of the amber mutant pre-mRNA with a high-score SF2/ASF motif (NH) was predominantly via exon 18 inclusion, whereas that of the missense mutant with a disrupted SF2/ASF motif (ML) was primarily via exon 18 skipping. Therefore, exon inclusion strongly correlates with a high-score SF2/ASF motif, and an in-frame nonsense mutation is neither necessary nor sufficient for exon skipping.

To determine whether the findings with *BRCA1* have general significance, it was examined whether point mutations in other genes can also disrupt ESEs. A database of 50 single-base substitutions known to cause exon skipping *in vivo* was analyzed. The wild-type and mutant sequences of each gene were compared using the above-mentioned motif-scoring matrices for four SR proteins and their respective threshold values. Remarkably, the search results indicated that more than half of these single-base substitutions reduced or eliminated at least one high-score motif for one or more of these SR proteins (Table 1). Over twice as many high-score motifs were reduced or eliminated by the mutations as were increased or created by them (43 vs. 21). This excess of high-score motifs in the wild-type set of sequences, compared to the mutant set, is statistically significant (p < 0.01, binomial exact test). Therefore, the aberrant exon skipping resulting from missense, nonsense, or translationally silent single-base substitutions is frequently, if not always, due to disruption of a critical ESE. Similar effects can be expected from small insertions or deletions within exons.

### Example 3. Methods for Examples 1 and 2

***BRCA1* DNA templates**. A portion of the wild-type human *BRCA1* gene was amplified by PCR from human genomic DNA (Promega) using primers T7P1 (5'-TAATACGACTCAC-TATAGGGAGATGCTCGTGTACAAGTTTGC) (SEQ ID NO.: 6.) and P6 (5'-AAGTACT-TACCTCATTCAGC) (SEQ ID NO.: 7.). The amplified DNA was then used as a template for three separate PCR amplifications to synthesize intron-truncated DNA fragments: the first PCR amplified exon 17 and the 5' part of intron 17 using primers T7P1 and P2 (5'-TAAGAAGCTAAAGAGCCTCACTCATGTGGTTTTATGCAGC) (SEQ ID NO.: 8); the second PCR amplified the 3' part of intron 17, exon 18, and the 5' part of intron 18 using primer P3 (5'-TGAGGCTCTTTAGCTTCTTA) (SEQ ID NO.: 9.) and P4 (5'-AGATAGAGAGGTCAGCGATTTGCA-ATTCTGAGGTGTTAAA) (SEQ ID NO.: 10.); the third PCR amplified the 3' part of intron 18 and exon 19 using primers P5 (5'-AATCGCTGACCTCTCTATCT) (SEQ ID NO.: 11) and P6. The three PCR products were then combined and amplified with primers T7P1 and P6. This overlap-extension PCR generated a *BRCA1* minigene (WT) with shortened introns but with otherwise natural intronic splicing signals, wild-type exons 17, 18, and 19, and a T7 bacteriophage promoter. The mutant *BRCA1* minigene NL was constructed by overlap-extension PCR with primers T7P 1 and P6 using as the template the products of two combined PCR amplifications of WT DNA: the first PCR was done with primers T7P1 and Pna (5'-CACACACAAACTAAGCATCTGC) (SEQ ID NO.: 12); the second PCR was done with primers Pns (5'-GCAGATGCTTAGTTTGTGTGTG) (SEQ ID NO.: 13.) and P6. The mutant *BRCA1* minigenes ML and NH were constructed similarly, except that the primers Pna and Pns were replaced by primers Pla (5'-CACACACAAACTTAGCATC-TGC) (SEQ ID NO.: 14.) and Pls (5'-GCAGATGCTAAGTTTGTGTGTG) (SEQ ID NO.: 15.), or primers Pha (5'-CACACACCT-ACTCAGCATCTGC) (SEQ ID NO.:16.) and Phs (5'-GCAGATGCTGAGTAGGTGTGTG) (SEQ ID NO.: 17), respectively.

***In vitro* transcription and splicing**. T7 runoff transcripts were uniformly labeled with ³²P-GTP or UTP, purified by denaturing PAGE, and spliced in HeLa cell nuclear extracts as described. Briefly, 20 fmol of ³²P-labeled, m⁷G(5')ppp(5')G-capped T7 transcripts were incubated in 25-µl splicing reactions containing 5µl of nuclear extract in buffer D, and 4.8 mM MgCl₂. After incubation at 30°C for 1 hr, the RNA was extracted and analyzed on 12% denaturing polyacrylamide gels, followed by autoradiography.

### Example 4. High-score motif analysis.

Wild-type or mutant exon sequences from the *BRCA1* gene and from the genes in Table 1 were analyzed with SR protein score matrices essentially as described in Liu et al., Nature Genet. 27:55-58 (2001), except for the use of slightly revised nucleotide frequency matrices and threshold values. The highest score for each SR protein was calculated for each sequence in a random-sequence pool, and the median of these high scores was set as the threshold value for that SR protein. The threshold values were: SF2/ASF heptamer motif - 1.956; SRp40 heptamer motif - 2.670; SRp55 hexamer motif - 2.676; SC35 octamer motif - 2.383.

Figure 13 shows the high-score SR protein motifs in *BRCA1* exon 18. The 78-nt sequence of wild-type exon 18 was searched with four nucleotide-frequency matrices derived from pools of functional enhancer sequences selected *in vitro* (Liu et al., Genes Dev. 12:1998-2012 (1998); (Liu et al., Mol. Cell Biol. 20:1063-1071 (2000).) The thresholds and maximal values are different for each SR protein. The G at position 6 (wild-type) is highlighted. The nonsense mutation that changes this G to a T only affects the first SF2/ASF motif, reducing the score from 2.143 to 0.079 (below the threshold).

Figure 14 illustrates that the *in vitro* splicing of *BRCA1* minigene transcripts reproduces the exon-skipping phenotype of a nonsense mutation. Wild-type and mutant *BRCA1* minigene transcripts were generated by PCR and *in vitro* transcription. An internal portion of each intron - away from the splice sites and branch site - was deleted to generate pre-mRNAs of adequate length for *in vitro* splicing. Wild-type (wt, lane 1) and nonsense mutant with low SF2/ASF score (NL, lane 2) radiolabeled transcripts were spliced in HeLa cell nuclear extract, and the products of the reaction were analyzed by denaturing PAGE and autoradiography.

Figure 15 illustrates that exon skipping correlates with the SF2/ASF enhancer motif score and not with reading frame disruption.

### Example 5. PNA-peptide targeted against BRCA1 exon 18

Figure 6 shows a PNA-peptide targeted against *BRCA1* exon 18. The PNA is positioned one nucleotide downstream of the mutation at exonic position +6 in *BRCA1* exon 18, so it can hybridize equivalently to wild-type and mutant sequences, the former one being used as a control. A 12-residue PNA length was used based on Tm, specificity, PNA sequence-composition empirical rules having to do with solubility, and cost considerations. A twenty amino acid peptide (RS)₁₀ was used as the peptide RS domain. The N-terminus of the peptide was linked to the C/3' end of the PNA. Two glycines were included as a linker between the PNA and the RS domain. The PNA-peptide was purified by HPLC and characterized by mass spectrometry. As controls, separate RS domain peptide and PNA molecules were obtained, as well as a PNA of unrelated sequence.

*In vitro* splicing experiments, under the conditions described above for the wild-type and mutant *BRCA1* exon 18 inclusion, were carried out in the presence of the PNA-peptide or the controls. (See Figure 7.) The products of splicing were analyzed by denaturing PAGE and autoradiography (top). The percentage of exon 7 inclusion was quantitated (bottom); the points on the curves are open symbols for the mutant, and solid symbols for the wild-type. Remarkably, the dose-response curves for each compound show that the PNA-peptide (BR PNA·RS) was effective at promoting exon 18 inclusion with the pre-mRNA harboring the patient nonsense mutation at position +6 (NL mut). The peptide alone (RS10 pep) had a slight inhibitory effect, whereas the PNA alone (BR1 PNA) had a slight stimulatory effect that was sequence-specific, since the control PNA of unrelated sequence (TAT1 PNA) had no effect. The slight but detectable positive effect of the PNA alone may reflect structural alterations of the pre-mRNA near the exon 18 3' splice site, which somehow facilitate binding of splicing components at the 3' splice site. In a separate experiment, dose-response curves with BR PNA·RS were carried out at different magnesium concentrations. (See Figure 8.) The C lanes show the input pre-mRNAs. At both magnesium concentrations, the PNA-peptide targeted to *BRCA1* increased the extent of inclusion of the mutant exon 18 in a dose-dependent manner.

### Example 6. Disruption of an SF2/ASF-dependent Exonic Splicing Enhancer Motif in SMN2 Exon 7

### SR protein ESE motifs in SMN1 and SMN2 exon 7.

*SMN1* exon 7 was analyzed using four sequence-motif matrices that predict functional ESEs recognized by the SR proteins SF2/ASF, SC35, SRp40 and SRp55. Only three motifs with scores above the thresholds for these proteins are present in *SMN1* exon 7: two for SF2/ASF and one for SC35 (Figure 9). Both the SC35 octamer and the SF2/ASF heptamer motifs (Figure 9), which overlap at the 5' end of *SMN1* exon 7, are disrupted in SMN2 by the C6T substitution (Figures 9 and 10).

To uncouple the effect of disrupting both the SF2/ASF and SC35 high-score motifs, the effect of substituting nucleotides G or A at position +6 of exon 7 (C6G and C6A) was first calculated. C6G reduces, but does not eliminate, the high scores of both SF2/ASF (3.76 to 2.18) and SC35 (3.87 to 2.95) motifs; C6A likewise results in a reduction in the SC35 high-score motif (3.87 to 2.59) but has a more severe effect on the SF2/ASF high-score motif, which drops below the threshold (3.76 to 1.26) (Figure 10). Using a semi-quantitative transient transfection assay, it was confirmed that C6G has essentially no effect on exon 7 inclusion, whereas C6A shows an intermediate phenotype (Figure 11, lanes 1, 3, 5, 7). Therefore, a strong correlation exists between the SR protein motif scores and exon 7 skipping. Skipping becomes significant in the absence of an SF2/ASF, but not an SC3 5, high-score motif, showing that the putative ESE is SF2/ASF-specific.

### A second-site suppressor mutation that reconstitutes a high-score SF2/ASF motif at the original position in SMN2 exon 7 fully restores exon inclusion.

If the motif-score matrices have predictive value, it should be possible to reconstruct a functional ESE within *SMN2* (equivalent to *SMN1* C6T) by introducing a second-site suppressor mutation that recreates a high-score motif at the same position, regardless of the precise sequence. To this end, a single A to G transition at position +11 of exon 7 (A11G) was introduced. This substitution places a highly conserved G at the sixth position of the SF2/ASF heptamer, replacing the non-consensus A (Figure 10, top). Because the SC35 high-score octamer spans positions 1 through 8 of the exon, it is unaffected by this change (Figure 10). The calculated motif scores for the A11G substitution, in conjunction with each of the four nucleotides at position 6, are shown in Figure 10. Notably, high-score SF2/ASF heptamers are recreated by the A11G substitution in both the C6T (*SMN2*) and C6A contexts (C6T/A11G and C6A/A11G, respectively). Accordingly, exon 7 inclusion was fully restored in the transient transfection assay only in the presence of an SF2/ASF high-score motif (Figure 11, lanes 2, 4, 6, 8). The fact that exon 7 was fully included even in the absence of an SC35 high-score motif (Figure 11, lane 4), and that an SC35 high-score motif was not sufficient to prevent exon skipping (Figure 11, lane 5), shows that SC35 does not play an essential role in mediating exon 7 inclusion.

### An SF2/ASF-dependent heptamer ESE is necessary and sufficient to promote exon inclusion in vitro.

To determine whether the SF2/ASF heptamer is a genuine enhancer, it was tested in a heterologous context, namely, exon 18 of *BRCA1* pre-mRNA. Inclusion of this exon in *BRCA1* mRNA depends on the integrity of an SF2/ASF-dependent ESE at positions +4 to +10 of the exon, such that only mutations that disrupt the ESE cause exon skipping, regardless of the mutation type. The SF2/ASF high-score motif in *BRCA1* exon 18 was substituted with the heptamer from *SMN1* exon 7, or with the corresponding sequence in *SMN2* (Figure 16a). Remarkably, the *SMN1* heptamer promoted exon 18 inclusion *in vitro* at levels comparable to wild-type *BRCA1* (Figure 16b, lanes 1 and 3), whereas the *SMN2*-derived heptamer was much less efficient, behaving similarly to a *BRCA1* natural exon-skipping mutant (Figure 16b, lanes 2 and 4) and reflecting the differences in SF2/ASF heptamer motif scores.

An *in vitro* system to study *SMN* pre-mRNA splicing was developed. As the *SMN1* and *SMN2* minigenes used for transfection assays are too large for *in vitro* studies, internal deletions in introns 6 and 7, and 3' truncations in the non-coding exon 8 were made. Although exon 8 is the last exon in the *SMN* genes, 10 nt were added which comprise a consensus 5' splice site at the 3' end of the minigenes to improve the overall splicing efficiency by exon definition. Several minigene transcript sets were tested, until a set that spliced *in vitro* with reasonable efficiency and faithfully reflected the *in vivo* splicing patterns was defined (Figure 17 and Methods below). The presence of the consensus 5' splice site at the 3' end greatly increased splicing efficiency (data not shown). An optimal set of four minigenes corresponding to *SMN1, SMN2,* and the A11G suppressor mutation in both context. (Figure 17a) was transcribed *in vitro* and spliced in HeLa cell nuclear extract. Exon 7-containing mRNAs were the predominant spliced product with the *SMN1* substrate (55% inclusion; Figure 17b, lane 1), whereas exon 7 skipping was favored with the C6T (*SMN2*) substrate (23% inclusion; Figure 17b, lane 2). In agreement with the transfection experiments (Figure 11), the A11G suppressor mutation in the *SMN2* context fully restored the inclusion levels observed with *SMN1* (Figure 17b, lane 4; 65% inclusion). Significantly, the same mutation in the *SMN1* context promoted exon inclusion with even higher efficiency than the wild type (Figure 17b, lane 3; 82% inclusion), consistent with the presence of a higher SF2/ASF motif score (6.03 vs. 3.76).

Finally, splicing of *SMN1* and *SMN2* pre-mRNAs in S100-complementation experiments was used to test the SR protein specificity of the ESEs. S100 extract is a post-nuclear, post-ribosomal fraction capable of supporting *in vitro* splicing only when complemented with one or more SR proteins. When the *SMN* pre-mRNAs were incubated in S 100 extract alone, spliced products were barely detectable (Figure 17b, lanes 5-8). Complementation with SF2/ASF gave splicing patterns comparable to those obtained with nuclear extract (Figure 17b, lanes 9-12). In particular, SF2/ASF promoted exon 7 inclusion with *SMN1* pre-mRNA (lane 9), but did so much less efficiently with *SMN2* pre-mRNA (lane 10). As with nuclear extract, the A11G suppressor mutation significantly increased the inclusion efficiency in both *SMN* gene contexts (lanes 11 and 12). The levels of exon 7 inclusion depended on the dose of SF2/ASF, and, at high concentrations, SF2/ASF promoted significant inclusion even in the *SMN2* context (data not shown). This result is consistent with the presence of a second SF2/ASF high-score motif downstream in the exon, in a region unaffected by the mutations (Figure 9). In contrast to SF2/ASF, recombinant SC35 failed to drive exon 7 inclusion (Figure 17b, lanes 13-16), even though it promoted splicing *via* exon 7 skipping (same lanes) and efficiently complemented S 100 extract with β-globin pre-mRNA (data not shown), again indicating that the SC35 motif in exon 7 is not a functional ESE.

### Example 7. Methods for Example 6

**Minigenes and Templates.** All *SMN* constructs were derived from pCITel. First, an *Xba* I site was inserted by site-directed mutagenesis at position 7170 (in intron 7) to generate pCI-SMNx-wt, using a Quickchange kit (Stratagene) with primers smnI7xbaF (AGATAAAAGGTTAATCTAGATCCCTACTAGAATTCTC) (SEQ ID NO.: 18) and smnI7xbaR (GAGAATTCTAGTAGGGATCTAGATTAACCTTTTATCT) (SEQ ID NO: 19). PCI-SMNx-wt was then used as a template to generate the following constructs (mutant bases underlined): pCISMNx-c6t (primers smnM6ctF, ATTTTCCTTACAGGGTTTTAGACAAAATCAAAAAGAAG (SEQ ID NO: 20) and smnM6ctR, CTTCTTTTTGATTTTGTCTAAAACCCTGTAAGGAAAAT) (SEQ ID NO: 21), pCISMNx-c6a (primers smnM6caF, ATTTTCCTTACAGGGTTTAAGACAAAATCAAAAAGAAG (SEQ ID NO: 22) and smnM6caR, CTTCTTTTTGATTTTGTCTTAAACCCTGTAAGGAAAAT) (SEQ ID NO: 23), pCISMNx-c6g (primers smnM6cgF, ATTTTCCTTACAGGGTTTGAGACAAAATCAAAAAGAAG (SEQ ID NO: 24) and smnM6cgR, CTTCTTTTTGATTTTGTCTCAAACCCTGTAAGGAAAAT) (SEQ ID NO: 25), pCISMNx-a11g (primers smnM11agF,
ATTTTCCTTACAGGGTTTCAGACGAAATCAAAAAGAAG (SEQ ID NO: 26) and smnM11agR, CTTCTTTTTGATTTCGTCTGAAACCCTGTAAGGAAAAT) (SEQ ID NO: 27), pCISMNx-c6t/a11g (primers smnM6ct/11agF,
ATTTTCCTTACAGGGTTTTAGACGAAATCAAAAAGAAG (SEQ ID NO: 28) and smnM6ct/11agR,
CTTCTTTTTGATTTCGTCTAAAACCCTGTAAGGAAAAT) (SEQ ID NO: 29), pCISMNx-c6a/a11g (primers smnM6ca/11agF,
ATTTTCCTTACAGGGTTTAAGACGAAATCAAAAAGAAG (SEQ ID NO: 30) and smnM6ca/11agR,
CTTCTTTTTGATTTCGTCTTAAACCCTGTAAGGAAAAT) (SEQ ID NO: 31), pCISMNx-c6g/a11g (primers smnM6cg/11agF,
ATTTTCCTTACAGGGTTTGAGACGAAATCAAAAAGAAG (SEQ ID NO: 32) and smnM6cg/11agR,
CTTCTTTTTGATTTCGTCTCAAACCCTGTAAGGAAAAT) (SEQ ID NO: 33).

Intron 6 was shortened by overlap-extension PCR to generate pCISMNxΔ6-wt. 5570 nt were deleted from position 1235 to the *Bcl* I site at position 6805. Two sets of PCR were performed with Pfu polymerase and pCISMNx-wt as template. The first PCR was carried out with primers CIF1
(AATTGCTAACGCAGTCAGTGCTTC) (SEQ ID NO: 34)and delta6-bclR (AATATGATCAGCAAAACAAAGTCACATAACTAC) (SEQ ID NO: 35), the second with primers smnΔ6-vrlp
(GTGACTTTGTTTTGCTGATCATATTTTGTTGAATAAAATAAG) (SEQ ID NO: 36) and CIR (AATGTATCTTATCATGTCTGCTCG) (SEQ ID NO: 37). The purified PCR products where then combined and reamplified with primers CIF1 and CIR. The final product was digested with *Xho* I and *Not* I and subcloned into pCISMNx-wt digested with the same enzymes. The mutations were introduced into pCISMNxΔ6-wt by subcloning a *Bcl* I-*Xba* I fragment containing part of intron 6, exon 7 and part of intron 7 from the full-length mutants into the corresponding sites of the new vector, to generate pCISMNxΔ6-c6t, pCISMNxΔ6-a11g, and pCISMNxΔ6-6/11. All the constructs were verified by direct sequencing. To obtain templates for *in vitro* transcription, the latter four plasmids were amplified with primers CIF2 (ACTTAATACGACTCACTATAGGCTAGCC) (SEQ ID NO: 38) and smn8-75+5'R (AAGTACTTACCTGTAACGCTTCACATTCCAGATCTGTC) (SEQ ID NO: 39). The final products contain a T7 promoter, exon 6 (124 nt), a shortened intron 6 (200 nt), wild-type or mutant exon 7 (54 nt), intron 7 (444 nt), and 75 nt of exon 8 followed by a consensus 5' ss. The *BRCA1*-derived constructs were generated by overlap-extension PCR using pBRCA1-WT as template. Primers T7P1(ref) and brSM1.R
(CAGTGTCCGTTCACACACATTGTCTGCATCTGCAGAATGAAAAACAC) (SEQ ID NO: 40) or brSM2.R
(CAGTGTCCGTTCACACACATTGTCTACATCTGCAGAATGAAAAACAC) (SEQ ID NO: 41) and primers brSM1.F
(GTGTTTTTCATTCTGCAGATGCAGACAATGTGTGTGAACGGACACTG) (SEQ ID NO: 42) or brSM2.F
(GTGTTTTTCATTCTGCAGATGTAGACAATGTGTGTGAACGGACACTG) (SEQ ID NO: 43) and P6(ref) were used in the first-step PCR, and T7P1 and P6 were used in the second step. The purified PCR products were directly used as transcription templates.

**Transfections and Reverse-Transcription-PCR (RT-PCR).** 293-HEK cells were transiently transfected by standard Ca₃(PO₄)₂ procedures with 10 µg of the indicated plasmids. 36 hours after transfection, total RNA was isolated using Trizol Reagent (Life Technologies) following the manufacturer's directions. One µg of DNAse-treated total RNA was used to generate first-strand cDNAs with oligo(dT) and Superscript II reverse transcriptase (Life Technologies), and cDNAs were amplified semi-quantitatively by 16 PCR cycles (94°C for 30 sec, 57.5°C for 30sec, 72°C for 90sec) using CIF2 and CIR primers in the presence of [α-³²P] dATP. The reaction products were resolved on 6% denaturing polyacrylamide gels.

### Example 8. In nitro transcription and splicing.

5' capped, T7 runoff transcripts from purified PCR products were uniformly labeled with [α-³²P] UTP, purified by denaturing PAGE, and spliced in HeLa cell nuclear or S 100 extracts, as described. Briefly, 10 fmol of transcript was incubated in 12.5-µl standard splicing reactions containing 3 µl of nuclear extract or 2 µl of S100 extract complemented with 4 pmol of recombinant SC35 or SF2/ASF. The MgCl₂ concentration was 2.4 mM for *BRCA1* transcripts and 1.6 mM for *SMN* transcripts. After incubation at 30°C for 4 hours, RNA was extracted and analyzed on 12% (*BRCA1*) or 8% (*SMN*) denaturing polyacrylamide gels, followed by autoradiography and phosphorimager analysis. Exon inclusion was calculated as a percentage of the total amount of spliced mRNAs, i.e., included mRNA × 100 /(included mRNA + skipped mRNA).

### Example 9. High-score motif analysis.

Exon sequences from *SMN1, SMN2,* and mutants thereof, were analyzed as described. For each SR protein, the highest score for each sequence in a pool of 30 random 20-mers was calculated, and the median of these high scores was set as the threshold value for that SR protein. The threshold values are: SF2/ASF heptamer motif, 1.956; SRp40 heptamer motif, 2.670; SRp55 hexamer motif, 2.676; SC35 octamer motif, 2.383. Scores below the thresholds are not considered significant.

Table 1 shows the alteration of enhancer motif scores by point mutations in human genes. A database of 50 single-base substitutions responsible for *in vivo* exon skipping in 18 human genes was analyzed with the score matrices for four SR proteins. Genes for which the mutation falls within, or creates, one or more high-score motifs are shown. Downward arrows denote a reduction or elimination of the motif score as a result of the mutation. Upward arrows denote a higher score in the mutant than in the wild-type. Sequence motifs for the same or for a different SR protein can overlap. Only the wild-type or mutant sequence motifs with scores greater than or equal to the threshold for the corresponding SR protein were considered. Fourteen mutations that do not fall within, or create, high-score motifs for SF2/ASF, SRp40, SRp55, or SC35 are not shown; they are: *ADA* R142X, *DYS* E1211X, *HPRTK55X, HPRT* G119X, *HPRT* G180X, *HPRT* G180E, *HPRT* G180V, *HPRT* E182X, *HPRT* E182K, *HPRT* D201V, *MNK* G1302R, *OAT W275X, PDH* G185G, *THY* R717X. Thirty-six mutations fell within, or created, one or more high-score motifs, and 27 of these mutations reduced or eliminated at least one high-score motif. There are over twice as many downward arrows (43) as upward arrows (21). N - nonsense mutation; M - missense mutation; S - synonymous mutation. The exon with the mutation, which is also the exon skipped during splicing, is indicated (column labeled Mut). The specific mutations are identified by the wild-type amino acid in the one-letter code, followed by the residue number in the protein sequence and the mutant amino acid (X denotes one of the three nonsense codons) as it would be in the absence of exon skipping (column labeled Sub.). Gene abbreviations: *ADA* - adenosine deaminase; *CFTR -* cystic fibrosis transmembrane conductance regulator; *DYS -* dystrophin; *FVIII -* factor VIII; *FACC -* Fanconi's anemia group C; *FBN1 -* fibrillin; *HEX-* β-hexosaminidase β subunit; *HMGCL -* hydroxymethylglutaryl-CoA lyase; *HPRT -* hypoxanthine phosphoribosyltransferase; *IDUA -* α-L-iduronidase; *MNK-* Menkes disease; *NF1 -* neurofibromatosis; *OAT -* ornithine δ-aminotransferase; *PBG -* porphobilinogen deaminase; *PDH -* pyruvate dehydrogenase; *PS -* protein S; *THY*- thyroglobulin; *WAS -* Wiskott-Aldrich syndrome.

**Table 1**

| **Gene** | **Mut.** | **Sub.** | **Exon** | **Type** | **SF2/ASF** | **SRp40** | **SRp55** | **SC35** |
|---|---|---|---|---|---|---|---|---|
| *CFTR* | E60X | G→T | 3 | N | | | | ↓ |
| CFTR | R75X | C→T | 3 | N | | | ↓ | |
| *CFTR* | R553X | C→T | 11 | N | | | | ↑ |
| *CFTR* | W1282X | G→A | 20 | N | ↓↓ | ↓ | | |
| *FVIII* | E1987X | G→T | 19 | N | ↓ | ↓ | | |
| *FVIII* | R2116X | C→T | 22 | N | | | ↑ | |
| *FACC* | R185X | C→T | 6 | N | ↓ | ↓↓ | | |
| *FBN1* | Y2113X | T→G | 51 | N | | ↓ | ↓ | |
| *HMDGCL* | E37X | G→T | 2 | N | | | ↑ | |
| *HPRT* | E30X | G→T | 2 | N | ↓ | | ↓↑ | |
| *HPRT* | E47X | G→T | 3 | N | | ↑ | | |
| *HPRT* | R51X | C→T | 3 | N | | ↓ | | |
| *HPRT* | C66X | T→A | 3 | N | | ↑ | ↓ | |
| *HPRT* | K103X | A→T | 3 | N | ↓ | | ↑ | ↓ |
| *HPRT* | L125X | T→G | 4 | N | | ↓ | | |
| *HPRT* | E197X | G→T | 8 | N | | ↑ | ↓ | |
| *HPRT* | Y198X | C→G | 8 | N | | ↑↓ | ↓ | |
| *IDUA* | Y64X | C→A | 2 | N | | | ↓ | |
| *MNK* | R645X | C→T | 8 | N | | ↓ | | |
| *NF1* | Y2264X | C→A | 37 | N | | ↓ | | |
| *NF1* | Y2264X | C→G | 37 | N | | ↓ | | |
| *OAT* | W178X | G→A | 6 | N | | ↓ | | ↓ |
| *PS* | S62X | C→G | 4 | N | | | | ↑ |
| *WAS* | Q99X | C→T | 3 | N | ↓ | | | |
| *ADA* | A215T | G→A | 7 | M | ↑↑ | ↑↑ | | |
| *HEX* | P404L | C→T | 11 | M | ↓ | | ↓ | |
| *HPRT* | G40V | G→T | 2 | M | ↑ | | ↑ | |
| *HPRT* | R48H | G→A | 3 | M | | | ↓ | |
| *HPRT* | A161E | C→A | 6 | M | ↓ | ↓↑ | | ↓ |
| *HPRT* | P184L | C→T | 8 | M | ↓ | ↑ | | ↓ |
| *HPRT* | D194Y | G→T | 8 | M | | ↑ | | ↓ |
| *HPRT* | E197K | G→A | 8 | M | | | ↓ | |
| *HPRT* | E197V | A→T | 8 | M | | | ↑ | |
| *FBN1* | I2118I | C→T | 51 | S | | | | ↑ |
| *HPRT* | F199F | C→T | 8 | S | | ↓ | ↓ | |
| *PBG* | R28R | C→G | 3 | S | | | ↓ | ↓ |

### Example 10. Specific targeting of double-stranded DNA by bis-PNA in vitro.

A gel-shift experiment shows that a PNA clamp binds specifically to double-stranded DNA, and that the binding is sensitive to mutations at the binding site. (See Figure 18.) As expected, the binding is sensitive to salt concentration and pH. For optimal binding under physiological conditions, a clamp in which C residues on the Hoogsteen strand are replaced by pseudoisocytosine is used. Clamps with this substitution, with or without various attached transcription activation domains, modulate γ-globin transcription after delivery to K562 or HeLa cells.

### Example 11. Expression of BRCA1 in lymphoblast cell lines.

PNA-RS chimeric molecules specific for *BRCA1* exon 18 (Figure 6), according to the invention, were introduced into transformed human lymphoblasts heterozygous for the mutant allele of *BRCA1* that causes skipping of exon 18. Figure 19 shows that spliced mRNAs arising from exon 18 inclusion or skipping are present at comparable levels in these cells, whereas homozygous wild-type control cells only express mRNA that includes exon 18. Delivery of the PNA-RS chimeric molecule results in a dose-dependent disappearance of the lower band and increase in the intensity of the upper band.

## Claims

1. A chimeric molecule comprising:
a base-pairing segment comprising naturally-occurring or modified purine and/or pyrimidine bases joined to a polypeptide moiety comprising a domain comprising
dipeptide repeats,
wherein the base-pairing segment binds specifically to a segment of pre-mRNA according to Watson-Crick rules of base pairing, and the polypeptide moiety modulates splicing of the segment of pre-mRNA.

2. A chimeric molecule according to Claim 1 wherein said base-pairing segment comprises a non-sugar or a modified sugar backbone.

3. A chimeric molecule according to Claim 2 wherein said modified sugar backbone comprises a 2'O-methyl ribose group.

4. A chimeric molecule according to Claim 2 wherein said non-sugar backbone comprises a peptide-nucleic acid (PNA) segment.

5. A chimeric molecule according to Claim 2 wherein said non-sugar backbone comprises morpholino groups.

6. A chimeric molecule according to Claim 1 wherein said chimeric molecule has a branched structure.

7. A chimeric molecule according to Claim 1 wherein said base-pairing segment comprises about six to about fifty bases.

8. A chimeric molecule according to Claim 7 wherein said base-pairing segment comprises about ten to about thirty bases.

9. A chimeric molecule according to Claim 1 wherein said polypeptide comprises about five to about fifty residues.

10. A chimeric molecule according to Claim 9 wherein said polypeptide comprises about fifteen to about thirty residues.

11. A chimeric molecule according to Claim 1 wherein said polypeptide moiety activates splicing of the segment of pre-mRNA.

12. A chimeric molecule according to Claim 1 wherein said domain comprises arginine-serine dipeptide repeats.

13. A chimeric molecule according to Claim 1 wherein said domain comprises arginine-glutamic acid dipeptide repeats.

14. A chimeric molecule according to Claim 13 wherein said domain comprises about five to about fifteen arginine-serine dipeptide repeats.

15. A chimeric molecule according to Claim 1 comprising a spacer sequence between said base-pairing segment and said polypeptide moiety.

16. A chimeric molecule according to Claim 15 wherein said spacer sequence comprises from about one to about twenty amino acid residues.

17. A chimeric molecule according to Claim 15 wherein said spacer sequence consists of at least one glycine.

18. A chimeric molecule according to Claim 1 wherein said modulation of splicing is modulation of alternative splicing.

19. A chimeric molecule according to Claim 1 wherein said segment of a pre-mRNA is an exon.

20. A chimeric molecule according to Claim 1 wherein said segment of a pre-mRNA is an intron.

21. A chimeric molecule according to Claim 1 wherein said segment of pre-mRNA comprises a mutation.

22. A chimeric molecule according to Claim 21 wherein said segment of pre-mRNA is an exon of SMN2.

23. A chimeric molecule according to Claim 22 wherein said exon of SMN2 is exon 7.

24. An in vitro method for modulating splicing of a segment of pre-mRNA by using a chimeric molecule comprising a base-pairing segment comprising naturally-occurring or modified purine and/or pyrimidine bases that binds specifically to the segment of pre-mRNA according to Watson-Crick rules of base pairing, joined to a polypeptide moiety comprising a domain comprising dipeptide repeats.

25. Use of a chimeric molecule for the manufacture of a medicament for the treatment of spinal muscular atrophy by modulating splicing of a segment of pre-mRNA, the chimeric molecule comprising a base-pairing segment comprising naturally-occurring or modified purine and/or pyrimidine bases that binds specifically to the segment of pre-mRNA according to Watson-Crick rules of base pairing, joined to a polypeptide moiety comprising a domain comprising dipeptide repeats.

26. The method of claim 24 or the use according to claim 25 wherein said chimeric molecule binds to said pre-mRNA segment from about 0 to about 300 residues from a splice site on said pre-mRNA segment.

27. The method or the use according to claim 24 or 25 wherein said chimeric molecule binds to an intron of said pre-mRNA segment.

28. The method or use according to claim 24 or 25 wherein said chimeric molecule binds to an exon of said pre-mRNA segment.

29. The method or use according to claim 24 or 25 wherein said modulation of splicing is modulation of the rate of splicing.

30. The method or use according to claim 24 or 25 wherein said modulation of splicing is modulation of alternative splicing.

31. The method or use according to Claim 30 wherein said modulation of alternative splicing increases the expression of a gene.

32. The method or use according to claim 24 or 25 wherein said modulation of splicing decreases the expression of a gene.

33. The method or use according to claim 32 wherein said modulation of splicing decreases the expression of an oncogene or a viral gene.

34. The method or use according to claim 24 or 25 wherein said modulation of splicing promotes inclusion of a target exon in a mRNA transcript.

35. The method or use according to claim 34 wherein an exonic splicing enhancer of said target exon is absent or inactive.

36. The method or use according to claim 35 wherein said exonic splicing enhancer of said target exon is absent or inactive due to a nonsense mutation, missense mutation, synonymous mutation, frameshift mutation, intra-exonic deletion, intra-exonic insertion or single-nucleotide polymorphism.

37. The method or use according to claim 36 wherein said target exon is an exon of SMN2.

38. The method or use according to claim 37 wherein said exon of SMN2 is exon 7.

39. The use according to claim 25 wherein said chimeric molecule is delivered in utero.

40. The method or use according to claim 24 or 25 wherein said splicing does not occur naturally.

## Patentansprüche

1. Chimäres Molekül umfassend:
ein Basenpaarungs-Segment, das natürlich vorkommende oder modifizierte Purin-und/oder Pyrimidinbasen umfasst, gebunden an einen Polypeptidrest, der eine Domäne umfasst, die Dipeptid-Wiederholungen umfasst, wobei das Basenpaarungs-Segment, gemäß den Watson-Crick-Regeln für die Basenpaarung, spezifisch an ein pre-mRNA-Segment bindet, und der Polypeptid rest das Spleißen des pre-mRNA-Segments moduliert.

2. Chimäres Molekül nach Anspruch 1, worin das Basenpaarungs-Segment ein Grundgerüst ohne Zucker oder ein modifiziertes Zuckergrundgerüst umfasst.

3. Chimäres Molekül nach Anspruch 2, worin das modifizierte Zuckergrundgerüst eine 2'O-Methylribosegruppe umfasst.

4. Chimäres Molekül nach Anspruch 2, worin das Grundgerüst ohne Zucker ein Peptid-Nukleinsäure (PNA)-Segment umfasst.

5. Chimäres Molekül nach Anspruch 2, worin das Grundgerüst ohne Zucker Morpholinogruppen umfasst.

6. Chimäres Molekül nach Anspruch 1, wobei das chimäre Molekül eine verzweigte Struktur hat.

7. Chimäres Molekül nach Anspruch 1, worin das Basenpaarungs-Segment etwa sechs bis etwa fünfzig Basen umfasst.

8. Chimäres Molekül nach Anspruch 7, worin das Basenpaarungs-Segment etwa zehn bis etwa dreißig Basen umfasst.

9. Chimäres Molekül nach Anspruch 1, worin das Polypeptid etwa fünf bis etwa fünfzig Reste umfasst.

10. Chimäres Molekül nach Anspruch 9, worin das Polypeptid etwa fünfzehn bis etwa dreißig Reste umfasst.

11. Chimäres Molekül nach Anspruch 1, worin der Polypeptidrest das Spleißen des pre-mRNA-Segments aktiviert.

12. Chimäres Molekül nach Anspruch 1, worin die Domäne Arginin-Serin-Dipeptid-Wiederholungen umfasst.

13. Chimäres Molekül nach Anspruch 1, worin die Domäne Arginin-Glutaminsäure-Dipeptid-Wiederholungen umfasst.

14. Chimäres Molekül nach Anspruch 13, worin die Domäne etwa fünf bis etwa fünfzehn Arginin-Serin-Dipeptid-Wiederholungen umfasst.

15. Chimäres Molekül nach Anspruch 1, das eine Spacer-Sequenz zwischen dem Basenpaarungs-Segment und dem Polypeptidrest umfasst.

16. Chimäres Molekül nach Anspruch 15, worin die Spacer-Sequenz von etwa einen bis etwa zwanzig Aminosäurerest(e) umfasst.

17. Chimäres Molekül nach Anspruch 15, worin die Spacer-Sequenz aus mindestens einem Glycin besteht.

18. Chimäres Molekül nach Anspruch 1, worin die Modulation des Spleißens eine Modulation des alternativen Spleißens ist.

19. Chimäres Molekül nach Anspruch 1, worin das pre-mRNA-Segment ein Exon ist.

20. Chimäres Molekül nach Anspruch 1, worin das pre-mRNA-Segment ein Intron ist.

21. Chimäres Molekül nach Anspruch 1, worin das pre-mRNA-Segment eine Mutation um fasst.

22. Chimäres Molekül nach Anspruch 21, worin das pre-mRNA-Segment ein Exon von SMN2 ist.

23. Chimäres Molekül nach Anspruch 22, worin das Exon von SMN2 das Exon 7 ist.

24. *In* vitro-Verfahren zur Modulation des Spleißens eines pre-mRNA-Segments unter Verwendung eines chimären Moleküls, das ein Basenpaarungs-Segment umfasst, das natürlich vorkommende oder modifizierte Purin- und/oder Pyrimidinbasen umfasst, das gemäß den Watson-Crick-Regeln für die Basenpaarung, spezifisch an das pre-mRNA-Segment bindet, verbunden mit einem Polypeptidrest, der eine Domäne umfasst, die Dipeptid-Wiederholungen umfasst.

25. Verwendung eines chimären Moleküls zur Herstellung eines Medikaments zur Behandlung von spinaler Muskelatrophie durch Modulation des Spleißens eines pre-mRNA-Segments, wobei das chimäre Molekül ein Basenpaarungs-Segment umfasst, das natürlich vorkommende oder modifizierte Purin- und/oder Pyrimidinbasen umfasst, das gemäß den Watson-Crick-Regeln für die Basenpaarung, spezifisch an das pre-mRNA-Segment bindet, verbunden mit einem Polypeptidrest, der eine Domäne umfasst, die Dipeptid-Wiederholungen umfasst.

26. Verfahren nach Anspruch 24 oder Verwendung nach Anspruch 25, worin das chimäre Molekül an das pre-mRNA-Segment von etwa 0 bis etwa 300 Reste von einer Spleißstelle des pre-mRNA-Segments bindet.

27. Verfahren oder Verwendung nach Anspruch 24 oder 25, worin das chimäre Molekül an ein Intron des pre-mRNA-Segments bindet.

28. Verfahren oder Verwendung nach Anspruch 24 oder 25, worin das chimäre Molekül an ein Exon des pre-mRNA-Segments bindet.

29. Verfahren oder Verwendung nach Anspruch 24 oder 25, worin die Modulation des Spleißens die Modulation der Spleißrate ist.

30. Verfahren oder Verwendung nach Anspruch 24 oder 25, worin die Modulation des Spleißens die Modulation des alternativen Spleißens ist.

31. Verfahren oder Verwendung nach Anspruch 30, worin die Modulation des alternativen Spleißens die Expression eines Gens erhöht.

32. Verfahren oder Verwendung nach Anspruch 24 oder 25, worin die Modulation des Spleißens die Expression eines Gens erniedrigt.

33. Verfahren oder Verwendung nach Anspruch 32, worin die Modulation des Spleißens die Expression eines Oncogens oder eines viralen Gens erniedrigt.

34. Verfahren oder Verwendung nach Anspruch 24 oder 25, worin die Modulation des Spleißens den Einschluss eines Ziel-Exons in ein mRNA-Transkript fördert.

35. Verfahren oder Verwendung nach Anspruch 34, worin ein Exon-Spleiß-Verstärker des Ziel-Exons abwesend oder inaktiv ist.

36. Verfahren oder Verwendung nach Anspruch 35, worin der Exon-Spleiß-Verstärker des Ziel-Exons aufgrund einer "Nonsense"-Mutation, "Missense"-Mutation, Synonym-Mutation, Rasterschubmutation, Deletion im Exon, Insertion im Exon oder eines Polymorphismus eines einzelnen Nukleotids abwesend oder inaktiv ist.

37. Verfahren oder Verwendung nach Anspruch 36, worin das Ziel-Exon ein Exon von SMN2 ist.

38. Verfahren oder Verwendung nach Anspruch 37, worin das Exon von SMN2 Exon 7 ist.

39. Verwendung nach Anspruch 25, worin das chimäre Molekül in den Uterus verabreicht wird.

40. Verfahren oder Verwendung nach Anspruch 24 oder 25, worin das Spleißen nicht natürlich vorkommt.

## Revendications

1. Molécule chimère comprenant :
un segment d'appariement de bases comprenant des bases purine et/ou pyrimidine naturelles ou modifiées, joint à un fragment polypeptidique comprenant un domaine comprenant
des répétitions dipeptidiques,
dans laquelle le segment d'appariement de bases se lie spécifiquement à un segment de pré-ARNm conformément aux règles de Watson-Crick de l'appariement de bases, et le fragment polypeptidique module l'épissage du segment de pré-ARNm.

2. Molécule chimère selon la revendication 1, dans laquelle ledit segment d'appariement de bases comprend une charpente non sucre ou une charpente de sucre modifié.

3. Molécule chimère selon la revendication 2, dans laquelle ladite charpente de sucre modifié comprend un groupe 2'O-méthyl-ribose.

4. Molécule chimère selon la revendication 2, dans laquelle ladite charpente non sucre comprend un segment d'acide peptido-nucléique (APN).

5. Molécule chimère selon la revendication 2, dans laquelle ladite charpente non sucre comprend des groupes morpholino.

6. Molécule chimère selon la revendication 1, laquelle molécule chimère a une structure ramifiée.

7. Molécule chimère selon la revendication 1, dans laquelle ledit segment d'appariement de bases comprend d'environ six à environ cinquante bases.

8. Molécule chimère selon la revendication 7, dans laquelle ledit segment d'appariement de bases comprend d'environ dix à environ trente bases.

9. Molécule chimère selon la revendication 1, dans laquelle ledit polypeptide comprend d'environ cinq à environ cinquante résidus.

10. Molécule chimère selon la revendication 9, dans laquelle ledit polypeptide comprend d'environ quinze à environ trente résidus.

11. Molécule chimère selon la revendication 1, dans laquelle ledit fragment polypeptidique active l'épissage du segment de pré-ARNm.

12. Molécule chimère selon la revendication 1, dans laquelle ledit domaine comprend des répétitions dipeptidiques d'arginine-sérine.

13. Molécule chimère selon la revendication 1, dans laquelle ledit domaine comprend des répétitions dipeptidiques d'arginine-acide glutamique.

14. Molécule chimère selon la revendication 13, dans laquelle ledit domaine comprend d'environ cinq à environ quinze répétitions dipeptidiques d'arginine-sérine.

15. Molécule chimère selon la revendication 1, comprenant une séquence d'espacement entre ledit segment d'appariement de bases et ledit fragment polypeptidique.

16. Molécule chimère selon la revendication 15, dans laquelle ladite séquence d'espacement comprend d'environ un à environ vingt résidus d'acides aminés.

17. Molécule chimère selon la revendication 15, dans laquelle ladite séquence d'espacement est constituée d'au moins une glycine.

18. Molécule chimère selon la revendication 1, dans laquelle ladite modulation d'épissage est une modulation d'épissage alternatif.

19. Molécule chimère selon la revendication 1, dans laquelle ledit segment d'un pré-ARNm est un exon.

20. Molécule chimère selon la revendication 1, dans laquelle ledit segment d'un pré-ARNm est un intron.

21. Molécule chimère selon la revendication 1, dans laquelle ledit segment de pré-ARNm comprend une mutation.

22. Molécule chimère selon la revendication 21, dans laquelle ledit segment de pré-ARNm est un exon de SMN2.

23. Molécule chimère selon la revendication 22, dans laquelle ledit exon de SMN2 est l'exon 7.

24. Procédé in vitro pour moduler l'épissage d'un segment de pré-ARNm par utilisation d'une molécule chimère comprenant un segment d'appariement de bases comprenant des bases purine et/ou pyrimidine naturelles ou modifiées, qui se lie spécifiquement au segment de pré-ARNm conformément aux règles de Watson-Crick de l'appariement de bases, joint à un fragment polypeptidique comprenant un domaine comprenant des répétitions dipeptidiques.

25. Utilisation d'une molécule chimère pour la fabrication d'un médicament destiné au traitement de l'amyotrophie spinale, par modulation de l'épissage d'un segment de pré-ARNm, la molécule chimère comprenant un segment d'appariement de bases comprenant des bases purine et/ou pyrimidine naturelles ou modifiées, qui se lie spécifiquement au segment de pré-ARNm conformément aux règles de Watson-Crick de l'appariement de bases, joint à un fragment polypeptidique comprenant un domaine comprenant des répétitions dipeptidiques.

26. Procédé selon la revendication 24 ou utilisation selon la revendication 25, dans lequel ladite molécule chimère se lie audit segment de pré-ARNm à un emplacement situé d'environ 0 à environ 300 résidus par rapport à un site d'épissage sur ledit segment de pré-ARNm.

27. Procédé ou utilisation selon la revendication 24 ou 25, dans lequel ladite molécule chimère se lie à un intron dudit segment de pré-ARNm.

28. Procédé ou utilisation selon la revendication 24 ou 25, dans lequel ladite molécule chimère se lie à un exon dudit segment de pré-ARNm.

29. Procédé ou utilisation selon la revendication 24 ou 25, dans lequel ladite modulation d'épissage est une modulation du taux d'épissage.

30. Procédé ou utilisation selon la revendication 24 ou 25, dans lequel ladite modulation d'épissage est une modulation d'épissage alternatif.

31. Procédé ou utilisation selon la revendication 30, dans lequel ladite modulation d'épissage alternatif augmente l'expression d'un gène.

32. Procédé ou utilisation selon la revendication 24 ou 25, dans lequel ladite modulation d'épissage diminue l'expression d'un gène.

33. Procédé ou utilisation selon la revendication 32, dans lequel ladite modulation d'épissage diminue l'expression d'un oncogène ou d'un gène viral.

34. Procédé ou utilisation selon la revendication 24 ou 25, dans lequel ladite modulation d'épissage favorise l'inclusion d'un exon cible dans un produit de transcription d'ARNm.

35. Procédé ou utilisation selon la revendication 34, dans lequel un amplificateur d'épissage exonique dudit exon cible est absent ou inactif.

36. Procédé ou utilisation selon la revendication 35, dans lequel ledit amplificateur d'épissage exonique dudit exon cible est absent ou inactif en raison d'une mutation non-sens, d'une mutation faux-sens, d'une mutation synonyme, d'une mutation par décalage de cadre, d'une délétion intra-exonique, d'une insertion intra-exonique ou d'un polymorphisme mononucléotidique.

37. Procédé ou utilisation selon la revendication 36, dans lequel ledit exon cible est un exon de SMN2.

38. Procédé ou utilisation selon la revendication 37, dans lequel ledit exon de SMN2 est l'exon 7.

39. Utilisation selon la revendication 25, dans laquelle ladite molécule chimère est délivrée in utero.

40. Procédé ou utilisation selon la revendication 24 ou 25, dans lequel ledit épissage ne se produit pas naturellement.
